# EUROPÄISCHE PATENTSCHRIFT

(11) **EP 1 420 012 B1**
(45) Veröffentlichungstag und Bekanntmachung des Hinweises auf die Patenterteilung: **02.11.2006**
(21) Anmeldenummer: 03103534.8
(22) Anmeldetag: 24.09.2003
(51) Int. Cl.: C07C 263/04, C07C 265/10

(54) **Verfahren zur Herstellung von aliphatischen Isocyanaten aus aromatischen Isocyanaten**
Process for the preparation of aliphatic isocyanates from aromatic isocyanates
Procédé de préparation d' isocyanates aliphatiques à partir d' isocyanates aromatiques

(30) Priorität: 18.11.2002 DE 10253803
(43) Veröffentlichungstag der Anmeldung: 19.05.2004
(73) Patentinhaber: Degussa AG, 40474 Düsseldorf (DE)
(72) Erfinder: Kohlstruck, Stephan, 48249 Dülmen (DE); Kreczinski, Manfred, 44652 Herne (DE); Lettmann, Christian, 48653 Coesfeld (DE); Stochniol, Guido, D-45721 Haltern an see (DE); Spyrou, Emmanouil, 46282 Dorsten (DE); Finke, Norbert, 45739 Oer-Erkenschwick (DE); Lomölder, Rainer, 48153 Münster (DE)

(56) Entgegenhaltungen:
- EP-A- 0 023 649
- EP-A- 0 324 190
- EP-A- 0 813 906
- EP-A- 0 814 098
- DE-A- 4 407 019

## Beschreibung

Die Erfindung betrifft ein Verfahren zur Herstellung von aliphatischen Isocyanaten aus aromatischen Isocyanaten über im Wesentlichen 3 Stufen. Insbesondere betrifft die Erfindung ein Verfahren zur Herstellung von Bis[4-Isocyanatocyclohexyl]methan (H₁₂MDI) aus Bis[4-Isocyanatophenyl]methan (MDI). In besonderer Weise richtet sich die Erfindung auf ein Verfahren zur Herstellung von H₁₂MDI mit einem Gehalt des trans-trans-Isomeren von < 30 %, bevorzugt von <20 %, besonders bevorzugt von 5 bis 15 %.

Der synthetische Zugang zu Isocyanaten kann über eine Reihe unterschiedlicher Routen erfolgen. Tetramethylxylylendiisocyanat (TMXDI) beispielsweise ist ein Produkt der klassischen Isocyanatsynthese (US 4 130 577), die auf der Umsetzung eines Alkylhalogenids mit einem Metallsalz der Isocyanursäure beruht. Das Verfahren liefert gute Ausbeuten, der Zwangsanfall an Metallchlorid ist jedoch ein Problem. Zudem müssen lange Reaktionszeiten in Kauf genommen werden. Insbesondere für den Labormaßstab geeignet sind die mit den Namen Curtius, Lossen und Hofmann verknüpften Nitren basierenden Umlagerungen, die auf Carbonsäuren als Isocyanatvorstufe beruhen [DE 199 22 996; W. Hentschel, *Chem. Ber.* **17**, 1284 (1884)]. Älteste und auch heute noch vorherrschende Variante zur großtechnischen Herstellung von Isocyanaten ist die so genannte Phosgenroute. Grundlage dieses Verfahrens ist Umsetzung von Aminen mit Phosgen als hochreaktivem und potentem Carbonylierungsmittel, wobei das resultierende Isocyanat mechanistisch betrachtet das Produkt einer Additions-Eliminierungs-Sequenz darstellt. Verfahrenstechnisch können zwei grundsätzlich Varianten unterschieden werden, Lösemittel- (DE 199 42 299, US4 922 005, EP 0 175 117, EP 0 716 079) und Gasphasenphosgenierung (US 4 847 408, EP 0 676 392, DE 198 00 529). Beim Lösemittelverfahren findet die Sequenz aus Phosgenaddition und HCl-Eliminierung im Lösemittel statt, im Fall der Gasphasenphosgenierung erfolgt der Prozess im Gasraum. Letztere nimmt als modernere Technologie verschiedene Vorteile für sich in Anspruch, darunter eine verbesserte Raum-ZeitAusbeute und die Möglichkeit, spezielle Isocyanate mit deutlich erhöhten Ausbeuten zugänglich zu machen (EP 0 764 633, EP 0 749 958). Nachteil beider Phosgenierungskonzepte ist der Einsatz von Phosgen, dass aufgrund seiner Toxizität und Korrosivität besonders hohe Anforderungen an seine Handhabung im industriellen Maßstab stellt.

Es hat deshalb eine Vielzahl von Vorschlägen gegeben, die Verwendung von Phosgen zur Herstellung von Isocyanaten in technischen Größenordnungen zu umgehen. Der Begriff Phosgen freies Verfahren wird häufig im Zusammenhang mit der Überführung von Aminen in Isocyanate unter Einsatz alternativer Carbonylierungsmittel, z.B. Harnstoff oder Dialkylcarbonat, benutzt (EP 0 018 586, EP 0 355 443, US4 268 683, EP0 990 644). Weitere Phosgen freie Technologien basieren auf der reduktiven Carbonylierung aromatischer Nitrogruppen bzw. der oxidativen Carbonylierung aromatischer Aminofunktionen als Initialschritt einer zum Isocyanat führenden Reaktionsfolge [DE-OS 23 43 826, DE-OS 26 35 490; F. J. Weigert, *J. Org. Chem.* **38** (1973), 1316; S. Fukuoka et al., *J. Org. Chem.* **49** (1984), 1458].

Von den so genannten Phosgen freien Verfahren hat sich bis dato allein die Harnstofftechnologie als kommerziell wettbewerbsfähige großtechnische Alternative zum Phosgenverfahren etablieren können (EP 0 018 586, EP 0 126 299, EP 0 126 300, EP 0 143 320, EP 0 355 443, EP 0 566 925, EP 0 568 782). Grundlage der so genannten Harnstoffroute ist die harnstoffvermittelte Überführung von Aminen in Isocyanate über einen zweistufigen Prozess. Im ersten Schritt werden ein Amin und ein Alkohol in Gegenwart von Harnstoff zu einem Urethan umgesetzt, welches im zweiten Schritt thermisch in Isocyanat und Alkohol gespalten wird (EP 0 355 443, EP 0 0568 782, EP 0 566 925, EP 0 524 554).

Eine alternative Möglichkeit zur Herstellung cycloaliphatischer Isocyanate besteht darin, zunächst cycloaliphatische Urethane mit einer oder mehreren Urethangruppen durch katalytische Hydrierung der entsprechenden ein- oder mehrkernigen aromatischen Urethane mit einer oder mehreren Urethangruppen und gegebenenfalls weiteren Substituenten herzustellen, und nachfolgend die cycloaliphatischen Urethane durch Abspaltung der Alkoholgruppen in die korrespondierenden cycloalipahtischen Diisocyanate zu überführen.

Es ist außerdem bekannt, dass bei der Hydrierung der genannten aromatischen Urethane aliphatische Urethane gebildet werden, bei denen cis-trans-Isomerie möglich ist. Im Falle der Hydrierung des MDU zum H₁₂MDU sind drei cis-trans-Isomere möglich: cis-trans-, cis-cis- und trans-trans-H₁₂MDU. Die Abspaltung der Alkoholgruppen eines H₁₂MDU-Isomerengemisches unter Bildung des Bis[4-Isocyanatocyclohexyl]methan führt zu einem Gemisch von H₁₂MDI-Isomeren, deren Verhältnis im Wesentlichen gleich dem Verhältnis der H₁₂MDU-Isomeren im Ausgangsprodukt ist.

Die anwendungstechnischen Eigenschaften des H₁₂MDI sind direkt mit dem Isomerenverhältnis, insbesondere dem Gehalt des 4,4'-trans-trans-Isomeren, verknüpft. Um eine gleich bleibende Produktqualität der aus dem H₁₂MDI hergestellten Polyurethane zu gewährleisten und aus Gründen der einfacheren Handhabbarkeit ist es von besonderer Bedeutung, dass das H₁₂MDI bei Raumtemperatur als homogene Flüssigkeit ohne Feststoffanteil vorliegt. Mit steigendem Gehalt des 4,4'-trans-trans-Isomeren sinkt die Temperatur, ab der sich im H₁₂MDI die ersten Feststoffpartikeln bilden. Produkte mit niedrigem 4,4'-trans-trans-Anteil sind daher in einem breiteren Temperaturbereich flüssig und besitzen deshalb einen erheblichen anwendungstechnischen Vorteil.

Wie bereits oben erwähnt ist das Isomerenverhältnis in einem H₁₂MDI, das aus H₁₂MDU durch Abspaltung der Alkoholgruppen hergestellt wurde, im Wesentlichem gleich dem Isomerenverhältnis im H₁₂MDU selbst. Wenn daher ein niedriger 4,4'-trans-trans-Anteil im H₁₂MDI erzielt werden soll, ist es besonders wirtschaftlich, wenn bei der Hydrierung des MDU ein H₁₂MDU mit niedrigem 4,4'-trans-trans-Anteil resultiert, dass dann direkt zu einem H₁₂MDI mit entsprechend niedrigem 4,4'-trans-trans-Anteil weiterverarbeitete werden kann.

Die Hydrierung von aromatischen Urethanen zu den entsprechenden cycloaliphatischen Urethanen erfolgt, wie aus den unten genannten Dokumenten hervorgeht, u. a. unter Verwendung von geträgerten Katalysatoren.

Das US-Patent 5 360 934 lehrt das gattungsgemäße Verfahren, wobei jedoch ein Rhodium enthaltender Trägerkatalysator verwendet wird. Als Aktivmetall kann auch Ruthenium anwesend sein. Nach der Lehre dieses Dokuments hängt die Katalysatoraktivität in erheblichem Umfang von der Modifikation des als Träger eingesetzten Aluminiumoxids ab. Hiernach sind Katalysatoren mit Delta-, Theta- und Kapa-Aluminiumoxid als Trägermaterial aktiver als ein Katalysator mit handelsüblichem Gamma-Aluminiumoxid als Trägermaterial.

Im Verfahren gemäß EP 0 813 906 lassen sich organische Verbindungen, darunter auch aromatische Verbindungen, in welchen mindestens eine funktionelle Gruppe an einem aromatischen Kern gebunden ist, unter Verwendung eines Ruthenium-Trägerkatalysators hydrieren. Der Katalysator kann als Aktivmetall zusätzlich zu Ruthenium andere Metalle aus der ersten, siebten oder achten Nebengruppe des Periodensystems enthalten. Das Trägermaterial weist eine BET-Oberfläche von höchstens 30 m²/g und einen mittleren Porendurchmesser von mindestens 50 nm auf. Gekennzeichnet ist der hier verwendete Katalysator zudem durch ein Verhältnis der Oberfläche des Aktivmetalls und Oberfläche des Katalysatorträgers von kleiner 0,05. Bei den makroporösen Trägermaterialien mit einem mittleren Porendurchmesser von vorzugsweise 500 nm bis ungefähr 50 µm handelt es sich bevorzugt um Aluminiumoxid und Zirkoniumdioxid. Einzelheiten zur Hydrierung von MDU zu HMDU sind diesem Dokument nicht zu entnehmen. Insbesondere wird die Hydrierung substituierter aromatischer Verbindungen, in denen entweder mindestens eine Hydroxy- oder eine Aminogruppe an einen aromatischen Kern gebunden ist, beschrieben. Im Gegensatz hierzu stellten sich die Erfinder der vorliegenden Anmeldung die Aufgabe, substituierte aromatische Urethane in cycloaliphatische Urethane mit niedrigem 4,4' -trans-trans-Anteil zu überführen.

Ein ähnliches Verfahren wie jenes der EP 0 813 906 lehrt die EP0 814 098: Als Trägermaterial für den trägergebundenen Ruthenium-Hydrierkatalysator werden hier solche Materialien verwendet, deren Porenvolumen zu 10 bis 50 % aus Makroporen mit einem Porendurchmesser im Bereich von 50 bis 10.000 nm und zu 50 bis 90 % aus Mesoporen mit einem Porendurchmesser im Bereich von 2 bis 50 nm gebildet wird. Die BET-Oberfläche des Trägers wird mit 50 bis 500 m²/g, insbesondere 200 bis 350 m²/g angegeben. Das Verhältnis der Oberfläche des Aktivmetalls und des Trägers soll weniger als 0,3, insbesondere weniger als 0,1, betragen. Angaben über die Aktivität derartiger Katalysatoren sowie das Isomerenverhältnis bei der Hydrierung von MDU zu H₁₂MDU lassen sich diesem Dokument nicht entnehmen.

Aus der EP 0 653 243 sind Katalysatoren bekannt, die sich zur Hydrierung von aromatischen Verbindungen eignen. Bei den darin aufgeführten Katalysatoren handelt es sich um Systeme, die durch Einbringen der gelösten Aktivkomponente in ein organisches Polymer hergestellt werden. Diese Mischung muss anschließend mit einem Trägermaterial vermengt werden, um abschließend geformt und temperaturbehandelt zu werden. Die so formulierte Herstellung des Katalysators gestaltet sich vergleichsweise aufwendig, da viele einzelne Teilschritte berücksichtigt werden müssen, die in Summe kostenintensiv sind, da eine Reihe von chemischen Zusätzen benötigt wird. Außerdem wird die Aktivkomponente homogen mit der Trägermasse vermischt, so dass es nur teilweise zur katalytischen Reaktion zur Verfügung steht.

Die DE-OS 26 39 842 beschreibt ein Verfahren zur Herstellung von cycloaliphatischen Urethanen durch Hydrierung aromatischer Urethane. Als Hydrierkatalysatoren werden Übergangsmetalle der VIII. Gruppe des Periodensystems eingesetzt, wobei Rhodium besonders bevorzugt ist. Beschrieben wird unter anderem auch die Hydrierung von Dimethyl 4,4'-methylendicarbanilat zu Dimethyl 4,4'-methylendicyclohexylcarbamat. Die Hydrierung wird in einem inerten Lösungsmittel, bevorzugt einem aliphatischen Alkohol, durchgeführt. Nachteilig an diesem Verfahren ist, dass die verwendeten Katalysatoren schnell an Aktivität verlieren und durch Spülen mit Schwefelsäure, Methanol und 2-Propanol nur teilweise regeneriert werden können. Außerdem sind keine Angaben zum 4,4'-trans-trans-Anteil im Produkt vorhanden und es findet sich auch keinerlei Hinweis darauf, dass dieser von Bedeutung ist.

In der DE-OS 44 07 019 wird ein Verfahren zur Herstellung von cycloaliphatischen Isocyanaten aus aromatischen Isocyanaten beschrieben. Das Verfahren umfasst drei Reaktionsschritte:
1. Umsetzung eines aromatischen Isocyanats mit einem Alkohol (Urethanisierung), bevorzugt Methanol. Die Urethanisierung erfolgt "in an sich bekannter Weise" (Anspruch 5).
2. Hydrierung des aromatischen Urethans in einem inerten Lösungsmittel mit Metallen der VIII. Gruppe des Periodensystems oder deren Verbindungen als Hydrierkatalysatoren, wobei Ruthenium besonders bevorzugt ist.
3. Abspaltung des Alkohols unter Freisetzung des Isocyanats "in an sich bekannter Weise" (Anspruch 5).

Als Beispiel wird die Herstellung von H₁₂MDI aus MDI angeführt. Dieses Beispiel beinhaltet die Hydrierung von Dimethyl 4,4'-methylendicarbanilat zu Dimethyl 4,4'-methylendicyclohexylcarbamat. Angaben zum 4,4' -trans-trans-Anteil des Produktes können dem Dokument nicht entnommen werden. Trägerkatalysatoren werden nur am Rande erwähnt.

Aus der EP 0 023 649 ist ein Verfahren zur Herstellung von aliphatischen Isocyanaten aus aromatischen Isocyanaten bekannt, das dadurch gekennzeichnet ist, dass zunächst ein aromatisches Isocyanat mit einem Lactam zur Reaktion gebracht wird und in einem nachfolgenden Schritt das lactamblockierte Isocyanat an einem Rhodiumkatalysator kernhydriert wird. Zur Gewinnung des freien aliphatischen Isocyanats wird das Lactam thermisch abgespalten. Unvorteilhaft ist, dass die lactamblockierten aromatischen Isocyanate bereits bei relativ niedrigen Temperaturen in Isocyanat und Lactam zurückspalten und dies zu Ausbeuteverlusten und zur Katalysatordesaktivierung führt. Zur Gewährleistung niedriger Reaktionstemperaturen werden daher ausschließlich Katalysatoren auf Rhodiumbasis eingesetzt, die aufgrund des zu Ruthenium vergleichsweise hohen Rhodiumpreises sehr teuer sind. Aus den in der EP 0 023 649 genannten Beispielen geht hervor, dass im Falle der Umsetzung von MDI zu H₁₂MDI ein Produkt mit einem 4,4'-trans-trans-Isomerenanteil von 32,1 % resultiert. Dieses Produkt ist laut Beschreibung, wie zu erwarten, bei Raumtemperatur nicht mehr vollständig flüssig, sondern enthält Kristalle.

Die EP 0 268 856 lehrt ein Verfahren zur Herstellung von Aralkyl- Mono- und Diurethanen durch säurekatalysierte Addition von Formaldehyd und Carbaminsäureestern an Aromaten. Die so hergestellten Produkte können entweder direkt zu aromatischen Isocyanaten gespalten werden oder zunächst kernhydriert und dann unter Freisetzung der aliphatischen Isocyanate gespalten werden. Bezüglich der Verteilung der cis-trans-Isomeren in den Produkten finden sich keine Hinweise. Insbesondere lässt sich aus dem Dokument kein Verfahren zur Herstellung von H₁₂MDU ableiten.

Aufgabe der vorliegenden Erfindung ist es, ein Verfahren zur Herstellung von aliphatischen Isocyanaten aus aromatischen Isocyanaten unter Verwendung eines rutheniumenthaltenen Trägerkatalysators zu finden, durch das die gewünschten cycloaliphatischen Isocyanate in hoher Selektivität gewonnen werden können. Eine weitere Aufgabe der Erfindung richtet sich auf die Bereitstellung eines Verfahrens zur Herstellung von H₁₂MDI, wobei der 4,4'-trans-trans-Isomerenanteil des H₁₂MDU weniger als 30 %, bevorzugt weniger als 20 %, besonders bevorzugt 5 bis 15%, betragen sollte. Eine weitere Aufgabe richtet sich darauf, dass trotz eines hohen Umsatzes ein niedriger 4,4'-trans-trans-Anteil erhalten bleibt. Gemäß einer weiteren Aufgabe sollte der im Verfahren eingesetzte Katalysator eine hohe Standzeit aufweisen und die Isomerenverteilung auch nach längerer Betriebsdauer im Wesentlichen unverändert belassen.

Gegenstand der Erfindung ist ein Verfahren zur Herstellung von cycloaliphatischen Isocyanaten aus den korrespondierenden aromatischen Isocyanaten, welche einen oder mehrere aromatische Ringe und eine oder mehrere, direkt und/oder indirekt an einem oder verschiedenen aromatischen Ringen gebundene Isocyanatgruppen aufweisen, umfassend im Wesentlichen die drei folgenden Stufen:
1. Urethanisierung des aromatischen Isocyanats,
2. Hydrierung des aromatischen Urethans mit Wasserstoff in Gegenwart eines geträgerten Katalysators, der als Aktivmetall Ruthenium alleine oder zusammen mit mindestens einem Metall der I., VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, und wobei der Katalysatorträger eine BET-Oberfläche im Bereich von größer 30 m²/g bis kleiner 70 m²/g aufweist und mehr als 50 % des Porenvolumens des Katalysatorträgers Makroporen mit einem Porendurchmesser von größer 50 nm und weniger als 50 % Mesoporen mit einem Porendurchmesser von 2 bis 50 nm sind,
3. Spaltung des hydrierten Urethans zum aliphatischen Isocyanat
wobei aromatische Isocyanate, ausgewählt aus Bis[4-isocyanatophenyl]methan, 2-Isocyanatophenyl-4'-isocyanatophenylmethan, 2-Isocyanatophenyl-2'-isocyanatophenylmethan (MDI) und mehrkernige methylenverbrückte Isocyanatophenyle (PMDI) sowie Gemische davon, 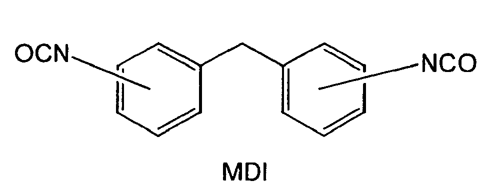 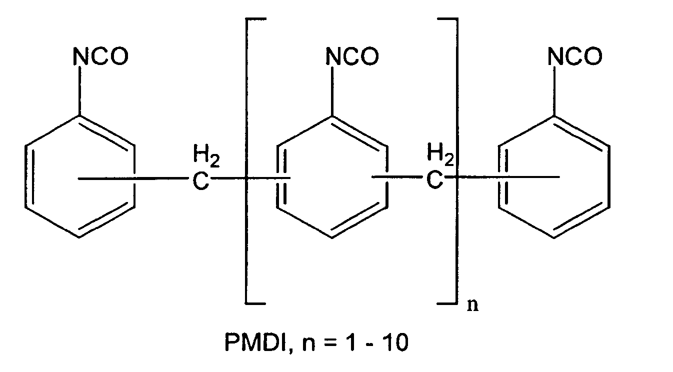 *4,4'-Diisocyanato-3,3'-dimethylphenylmethan, 2,4'-Diisocyanato-3,3'-dimethylphenylmethan, 2,2'-Diisocyanato-3,3'-dimethylphenylmethan* sowie Gemische davon, 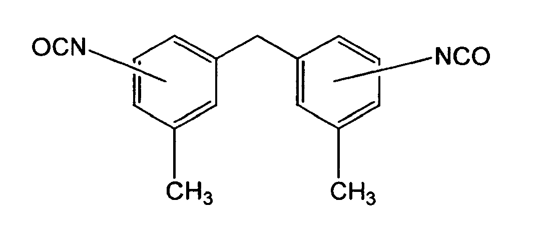 1,2-Diisocyanatobenzol, 1,3-Diisocyanatobenzol und 1,4-Diisocyanatobenzol sowie Gemische davon, 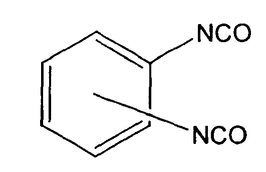 2,4-Diisocyanatotoluol, 2,6-Diisocyanatotoluol sowie deren Mischungen, 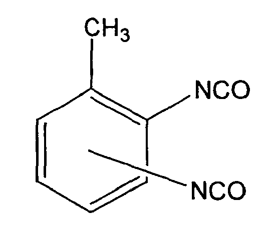 1,6-Diisocyanatonaphthalin, 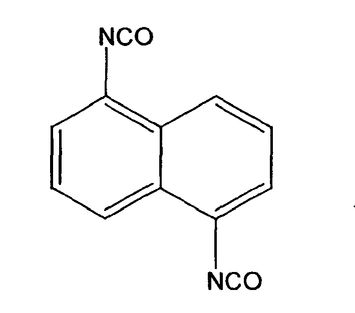 MXDI und TMXDI, 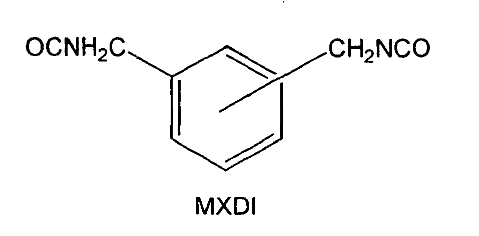 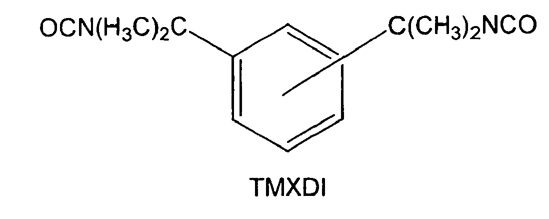 , eingesetzt werden.

Die Unteransprüche richten sich auf bevorzugte Ausführungsformen des erfindungsgemäßen Verfahrens.

Im Hinblick auf den zuvor eingehend gewürdigten Stand der Technik, insbesondere der EP 0 814 098, war es überraschend, dass ein Katalysatorträger mit einer spezifischen Oberfläche im Bereich von größer 30 m²/g bis kleiner 70 m²/g im gattungsgemäßen Verfahren besonders wirksam ist, wenn mehr als 50 % des Porenvolumens aus Makroporen und weniger als 50 % des Porenvolumens aus Mesoporen gebildet wird. Wesentlich ist somit nicht die BET-Oberfläche allein oder die Porenverteilung allein, sondern die Kombination dieser beiden Merkmale. Schließlich unterscheidet sich der im Verfahren der vorliegenden Erfindung gemäß zu verwendende Katalysator in prinzipieller Weise von dem in der EP 0 813 906 genannten Katalysator, weil der Katalysatorträger im vorbekannten Verfahren zwar makroporös ist, die BET-Oberfläche jedoch höchstens 30 m²/g und vorzugsweise höchstens 15 m²/g betragen soll. Das Verhältnis der Oberfläche des Aktivmetalls und des Katalysatorträgers liegt im Bereich von 0,01 bis 0,5, insbesondere 0,03 bis 0,3. Überraschender Weise führt auch ein geringes Oberflächenverhältnis des Aktivmetalls, bestimmt durch CO-Chemisorption, und des Katalysatorträgers, bestimmt nach BET, von 0,03 bis 0,06 bei erfindungsgemäß zu verwendenden Katalysator unter milden Hydrierbedingungen zu einer hohen Katalysatoraktivität.

Es wurde überraschenderweise gefunden, dass durch das erfindungsgemäße Verfahren in Verbindung mit den erfindungsgemäß eingesetzten Katalysatoren Isocyanate mit geringen trans-trans-Isomerenanteilen von weniger als 30 % erhalten werden. Insbesondere eignet sich das Verfahren auch zur Herstellung von Hydrierprodukten mit einem trans-trans-Isomerenanteil von kleiner 20 %, insbesondere von 5 bis 15 %, aus verbrückten zweikernigen Ausgangsprodukten, wie beispielsweise im nächsten Abschnitt genannt.

Das Verfahren eignet sich insbesondere zur Herstellung von Bis[4-Isocyanatocyclohexyl]methan (H₁₂MDI) mit einem trans-trans-Isomerenanteil von < 30 %, bevorzugt von < 20 %, besonders bevorzugt von 5 bis 15 %.

Mit dem erfindungsgemäßen Verfahren können aromatische Isocyanate jeglicher Art zu den entsprechenden cycloaliphatischen Isocyanaten umgesetzt werden. Dabei können die aromatischen Isocyanate einkernige oder mehrkernige aromatische Verbindungen sein. Vorzugsweise sind die aromatischen Verbindungen ein- und zweikernige aromatische Isocyanate oder Diisocyanate oder Triisocyanate. Die aromatischen Isocyanate können an dem oder den aromatischen Kernen oder/und an der Isocyanatgruppe substituiert sein, beispielsweise durch einen oder mehrere Alkyl- und/oder Alkoxyreste, vorzugsweise C₁₋₂₀-Alkyl-und/oder C₁₋₂₀-Alkoxyreste. Besonders bevorzugte Substituenten sind C₁₋₁₀-Alkylreste, insbesondere Methyl-, Ethyl-, Propyl-, Isopropyl-, Butyl-, Isobutyl-, tert.-Butylreste; unter den Alkoxyresten sind die C₁₋₈-Alkoxyreste, insbesondere Methoxy, Ethoxy, Propoxy und Butoxy bevorzugt. Der oder die aromatischen Kerne sowie die Alkyl- und Alkoxyreste können gegebenenfalls durch Halogenatome, insbesondere Fluoratome, substituiert sein oder andere geeignete inerte oder hydrierbare Substituenten aufweisen.

Das aromatische Isocyanat kann auch mehrere aromatische Kerne aufweisen, die über einen zweiwertigen Kohlenwasserstoffrest, wie eine Methylengruppe oder Ethylengruppe verknüpft sind, wobei einer oder beide aromatischen Kerne eine weitere Isocyanatgruppe und/oder eine C₁-bis C₃-Alkyl- oder Alkoxygruppe aufweisen können. Der verknüpfende Rest kann einen oder mehrere Alkylsubstituenten, insbesondere C₁₋₂₀-Alkylreste, bevorzugt einen oder mehrere Methyl-, Ethyl-, n- oder iso-Propyl-, n- oder sek.-Butyl- oder tert.-Butylreste aufweisen.

Besonders bevorzugt wird MDI eingesetzt.

Im Rahmen des erfindungsgemäßen Verfahren wird bevorzugt ein Startmaterial eingesetzt, das die Standzeit des Katalysators nicht beeinträchtigt. Es hat sich als vorteilhaft herausgestellt, wenn keine phosphor-, schwefel und/oder chlorhaltigen Verbindungen im Startmaterial vorhanden sind.

### URETHANISIERUNG:

Die Umsetzung von Isocyanaten mit Alkoholen ist bekannt und wurde in der Fachliteratur bereits häufig beschrieben (z. B. Ullmann's Enzyklopädie der technischen Chemie, 4. Auflage, Band 19, Seiten 310 bis 340).

Die Reaktion kann in Reinsubstanz erfolgen, in der Regel werden aber aufgrund der geringeren Viskosität Lösungsmittel eingesetzt. Als Lösungsmittel kommen alle Flüssigkeiten in Frage, die sich gegenüber den Reaktionspartnern als inert erweisen, also beispielsweise Ketone wie Aceton und Methylethylketon, Aromaten wie z. B. Toluol und Xylole, Amide wie Dimethylformamid und N-Methylpyrrolidon, Ether wie Diethylether, Dioxan und Tetrahydrofuran und Ester wie Ethylacetat, Butylacetat und Methoxypropylacetat. Selbstverständlich sind auch Mischungen von Lösungsmitteln möglich.

Die in Frage kommenden Lösemittel sollten möglichst wasserfrei sein (Wassergehalt < 0,1 Gew.-%).

Als Alkohole können alle primären, sekundären oder tertiären Monoalkohole eingesetzt werden, wie z. B. Methanol, Ethanol, Propanol, Isopropanol, n-Butanol, 2-Butanol, sek-Butanol, tert.-Butanol, n-Pentanol, 2-Pentanol, 3-Pentanol, isoPentanol, Neo-pentylalkohol, Hexanol, Cyclohexanol und Ethylhexanol. Selbstverständlich sind auch Mischungen von Alkoholen möglich.

Das Verhältnis von Isocyanatgruppen zu Alkoholgruppen wird so eingestellt, dass auf jede Isocyanatgruppe mindestens eine Alkoholgruppe kommt. Im Regelfall wird aber ein Überschuss an Alkoholen eingesetzt. Der Überschuss kann bis zu dem 100-fachen des Isocyanat-Äquivalenten betragen. In diesem Fall fungiert die Alkoholkomponente zusätzlich als Lösungsmittel.

Die Umsetzung von Isocyanaten, insbesondere von MDI, und Alkoholen, insbesondere mit n-Butanol, wird im Regelfall bei Temperaturen von 20 bis 160 °C durchgeführt, bevorzugt bei 40 bis 120 °C bei Normaldruck.

Die Reaktionszeit - im Regelfall zwischen 20 Minuten und 10 Stunden - kann mit Hilfe von Parametern, wie Temperatur, Monomerkonzentration und Monomerreaktivität, beeinflusst werden.

Außerdem lässt sich die Reaktion auch mit Katalysatoren beschleunigen, wie sie in der Urethanchemie schon lange bekannt sind. In Frage kommen metallhaltige Katalysatoren, wie z. B. Dibutylzinndilaurat und Zinkoctoat und tertiäre Amine wie z. B. Triethylamin und 1,4-Diazabicyclo-(2,2,2)-octan.

Als Reaktionsgefäße kommen Rührkessel, Rührkesselkaskaden, kontinuierlich oder diskontinuierlich betrieben, Strömungsrohre oder Extruder in Frage. Letztere eignen sich vor allem für den Fall, dass kein Lösungsmittel eingesetzt wird. Die Wahl eines geeigneten Extruders ist dem Fachmann geläufig (vgl. "Schneckenmaschinen in der Verfahrenstechnik", H. Hermann, Springer Verlag, Berlin, Heidelberg, New York 1972).

### HYDRIERUNG:

Die zur Hydrierung besonders bevorzugten aromatischen Urethane sind die folgenden aufgezählten und durch die Formeln beschriebenen Verbindungen:
Dialkyl 4,4'-methylendicarbanilat, Dialkyl 2,4'-methylendicarbanilat, Dialkyl 2,2'-methylendicarbanilat und mehrkernige methylenverbrückte Alkyl-Carbanilate (PMDU) sowie Gemische davon, 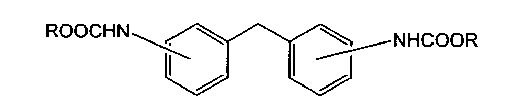 R = C₁-C₆-Alkyl, bevorzugt n-Butyl 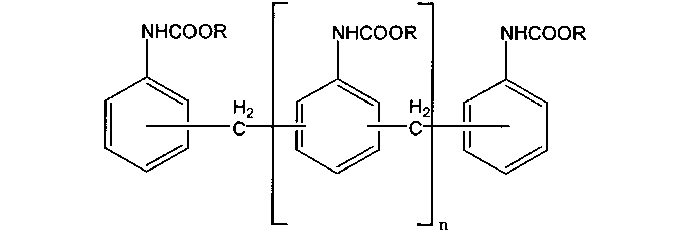 PMDU, R = C₁-C₆-Alkyl, bevorzugt n-Butyl, n = 1 - 10
Dialkyl 4,4'-methylen-3,3'-dicarbanilat, Dialkyl 2,4'-methylen-3,3'-dicarbanilat, Dialkyl 2,2'-methylen-3,3'-dicarbanilat sowie Gemische davon, 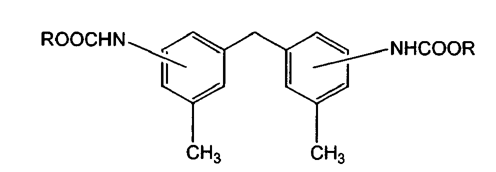 R = C₁-C₆-Alkyl, bevorzugt n-Butyl
Dialkyl 1,2-Phenyldicarbamat, Dialkyl 1,3-Phenyldicarbamat und Dialkyl 1,4-Phenyldicarbamat sowie Gemische davon, 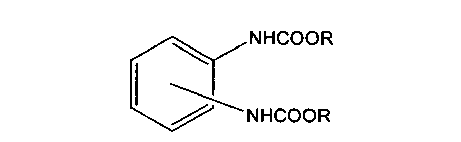 R = C₁-C₆-Alkyl, bevorzugt n-Butyl
Dialkyl 2,4-Toluoldicarbamat, Dialkyl 2,6-Toluoldicarbamat sowie deren Mischungen, 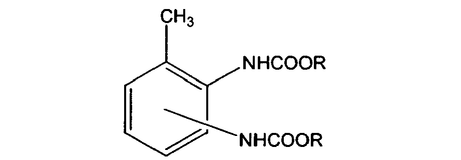 R = C₁-C₆-Alkyl, bevorzugt n-Butyl
Dialkyl 1,6-Naphthalindicarbamat 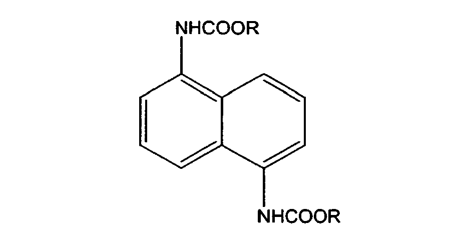 R = C₁-C₆-Alkyl, bevorzugt n-Butyl
die zum so genannten MXDI und TMXDI korrespondierenden Urethane, 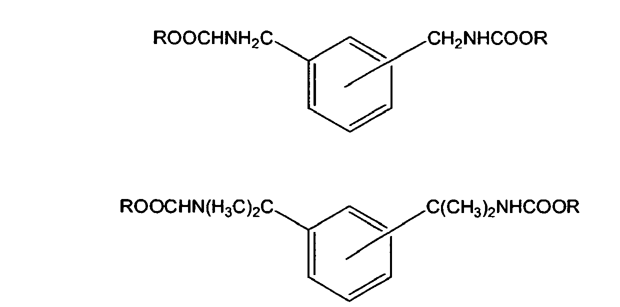 (R = Alkyl, bevorzugt n-Butyl)

Bevorzugte Verbindungen sind Dialkyl 4,4'-(C₁ bis C₄)alkan-dicarbanilat und/oder ein 2,4'-und/oder 2,2'-Isomeres oder Gemische hiervon, besonders bevorzugt Dibutyl 4,4'-methylendicarbanilat oder ein Isomeres oder ein Gemisch (MDU). Insbesondere wird Dibutyl 4,4'-methylendicarbanilat zu Dibutyl 4,4'methylendicyclohexylcarbamat mit einem trans-trans-Isomerenanteil von < 30 %, bevorzugt von < 20 %, besonders bevorzugt von 5 bis 15 % hydriert.

Die erfindungsgemäß zu verwendenden Trägerkatalysatoren können technisch hergestellt werden durch Auftragen von Ruthenium und gegebenenfalls mindestens einem Metall der I., VII. oder VIII. Nebengruppe auf einen geeigneten Träger. Die Auftragung kann durch Tränken des Trägers in wässrigen Metallsalzlösungen, wie Rutheniumsalzlösungen, durch Aufsprühen entsprechender Metallsalzlösungen auf den Träger oder durch andere geeignete Verfahren erreicht werden. Als Salze zur Herstellung der Rutheniumsalzlösungen sowie Lösungen von Metallsalzen von Elementen der 1-, VII- oder VIII- Nebengruppe eignen sich die Nitrate, Nitrosylnitrate, Halogenide, Carbonate, Carboxylate, Acetylacetonate, Chlorkomplexe, Nitrokomplexe oder Aminkomplexe der entsprechende Metalle; bevorzugt sind Nitrate und Nitrosylnitrate.

Bei Katalysatoren, die neben Ruthenium noch weitere Metalle auf den Träger aufgetragen enthalten, können die Metallsalze bzw. Metallsalzlösungen gleichzeitig oder nacheinander aufgebracht werden.

Die mit einem Rutheniumsalz bzw. zusätzlich weiteren Metallsalzlösungen beschichteten bzw. getränkten Träger werden getrocknet, vorzugsweise bei Temperaturen zwischen 100 und 150 °C, und wahlweise bei Temperaturen zwischen 200 und 600 °C kalziniert. Anschließend werden de beschichteten Träger durch Behandlung der beschichteten Träger in einem Gasstrom, der freien Wasserstoff enthält, bei Temperaturen zwischen 30 und 600 °C, vorzugsweise zwischen 150 und 400 °C aktiviert. Der Gasstrom besteht vorzugsweise aus 50 bis 100 Vol.-% H₂ und 0 bis 50 Vol.-% N₂.

Werden auf den Trägern neben Ruthenium noch ein oder mehrere andere Metalle der I., VII. oder VIII. Nebengruppe aufgetragen, und erfolgt das Auftragen nacheinander, so kann der Träger nach jedem Auftragen bzw. Tränken bei Temperaturen zwischen 100 und 150 °C getrocknet werden und wahlweise bei Temperaturen zwischen 200 und 600 °C kalziniert werden. Dabei kann die Reihenfolge, in der die Metallsalzlösungen aufgetragen werden, beliebig gewählt werden.

Gemäß einer bevorzugten Ausführung erfolgt die Beschichtung des Trägers durch Aufsprühen einer Metallsalzlösung bei erhöhter Temperatur, insbesondere oberhalb 50 °C und besonders bevorzugt bei 80 bis 150 °C, so dass bereits während der Beschichtung das Lösungsmittel zumindest teilweise verdampft wird und die Eindringtiefe der katalytisch wirksamen Metalle limitiert wird. Vorzugsweise liegt die Eindringtiefe im Bereich von 5 bis 250 µm, insbesondere 10 bis 150 µm und besonders bevorzugt bei 50 bis 120 µm.

Die Rutheniumsalzlösung und gegebenenfalls weitere Metallsalzlösungen werden in einer solchen Menge auf den oder die Träger aufgebracht, dass 0,01 bis 20 Gew.-%, bezogen auf das Gesamtgewicht des Katalysators, an Ruthenium und gegebenenfalls anderen Metallen der 1., VII. oder VIII. Nebengruppe auf den Träger aufgebracht werden. Vorzugsweise beträgt die Menge an Aktivmetallen 0,2 bis 15 Gew.-%, insbesondere etwa 0,2 bis 3 Gew.-%, wobei der Rutheniumgehalt den Gehalt der anderen Metalle zweckmäßiger Weise übersteigt.

Die Trägermaterialien der erfindungsgemäß zu verwendenden Katalysatoren weisen eine spezifische BET-Oberfläche (bestimmt nach DIN 66 131 mit N₂) im Bereich von größer 30 m²/g und kleiner 70 m²/g auf.

Der Träger enthält Makroporen mit einem Porendurchmesser von größer 50 nm. Der Durchmesser der Makroporen liegt insbesondere im Bereich von 50 bis 50 000 nm, vielfach aber im Bereich von 50 bis 10 000 nm. So weit der Träger auch Mesoporen umfasst, werden hierunter Poren im Bereich von 2 bis 50 nm verstanden. Mindestens 50 % des Porenvolumens wird aus Makroporen und weniger als 50 % aus Mesoporen gebildet. Bevorzugte Träger enthalten Makroporen in einer Menge von 55 bis 85 % des Porenvolumens, und 15 bis 45 % des Porenvolumens nehmen Mesoporen ein. In besonders bevorzugten Trägern nehmen Mesoporen etwa 25 bis 45 % des Porenvolumens ein und Makroporen den Rest des Porenvolumens. Mikroporen mit einem Porendurchmesser von kleiner 2 nm sind, sofern anwesend, nur in einer Menge von weniger als 10 % des Porenvolumens, insbesondere weniger als 1 % anwesend.

Die Modifikation des Trägers kann einheitlich oder gemischt sein, so dass die Porenverteilung monomodal, bimodal oder trimodal sein kann.

Prinzipiell sind alle bekannten Trägermaterialien für Hydrierkatalysatoren verwendbar, so weit sie die anspruchsgemäße BET-Oberfläche, Porengröße und Porenverteilung aufweisen. Geeignet sind oxidische, silikatische und nitridische Träger und zwar in ein- oder mehrphasiger kristalliner oder röntgenamorpher oder gemischter Struktur.

Die Träger können ferner in bekannter Weise mit Alkali- oder/und Erdalkaliverbindungen und/oder mit Metallen der Lanthanidreihe modifiziert sein.

Beispielhafte Träger sind Oxide aus der Reihe Al₂O₃, TiO₂, ZrO₂, SiO₂, MgO und ZnO, ferner Mischoxide, wie Spinelle, z. B. MgAl₂O₄. Auch Alumosilikate und Aktivkohle sind geeignet, sofern diese Träger die anspruchsgemäße Eigenschaftskombination aufweisen. Besonders bevorzugt sind die Oxide Al₂O₃ und TiO₂.

Die Hydrierung wird bei einer Temperatur im Bereich von 20 bis 250 °C, insbesondere bei unter 200 °C und einem wirksamen H₂-Partialdruck, im Bereich von etwa 1 bis 30 MPa, bevorzugt unter 15 MPa, in einem kontinuierlich oder diskontinuierlich zu betreibenden Suspensions- oder Festbett-Hydrierreaktor durchgeführt. Die Aktivität der erfindungsgemäßen Katalysatoren ermöglicht es, die Hydrierung unter milden Bedingungen, insbesondere bei einer Temperatur im Bereich von 50 bis 150 °C, insbesondere 70 bis 120 °C und einem H₂-Druck im Bereich von 3 bis 15 MPa durchzuführen, so dass technisch weniger aufwendige Reaktoren verwendet werden können, wodurch die Wirtschaftlichkeit des Verfahrens steigt.

Ein weiterer wirtschaftlicher Vorteil, der sich aus den milden Hydrierbedingungen ergibt, ist eine Steigerung der Gesamtausbeute des Verfahrens. Dies ist vor allem darauf zurückzuführen, dass mit steigender Temperatur zunehmend Rückspaltung des Urethans in Isocyanat und Alkohol erfolgt. Durch die anschließende Hydrierung der ungeschützten Isocyanatgruppe kommt es zur Bildung unerwünschter Nebenprodukte, die vom Produkt abgetrennt werden müssen und so einen Ausbeuteverlust bedingen.

Die Hydrierung kann in An- oder Abwesenheit eines geeigneten Lösungsmittels durchgeführt werden. Bevorzugt ist ein Lösungsmittel anwesend, und zwar in einer Menge von etwa 10 bis 90 Gew.-%, bezogen auf die Lösung des zu hydrierenden aromatischen Urethans.

Geeignete Lösungsmittel sind beispielhaft: primäre, sekundäre und tertiäre ein- oder mehrwertige Alkohole, wie Methanol, Ethanol, n- und i-Propanol, n-, sek.-, und tert.-Butanol, Ethylenglykol, Ethylenglykolmono(C₁-C₄)alkylether; lineare Ether, wie Ethylenglykoldi(C₁-C₃)alkylether; cyclische Ether, wie Tetrahydrofuran und Dioxan, Alkane, wie n- und iso-Alkane mit 4 bis 12 C-Atomen, z. B. n-Pentan, n-Hexan und Isooctan, und zyklische Alkane, wie Cyclohexan und Dekalin.

Lösungsmittel kann auch das Hydrierprodukt selbst, also ein cycloaliphatisches Urethan sein.

In einer bevorzugten Ausführungsform des Verfahrens wird ein Gemisch aus zwei oder mehr Lösungsmitteln eingesetzt, dass zusammengesetzt ist aus Alkoholen und Ethern, wobei der Alkohol insbesondere dem im Urethan enthalten Alkoholrest entspricht, bevorzugt n-Butanol. Bevorzugter Ether ist THF. Es wurde überraschend gefunden, dass der Alkoholzusatz nicht nur zu einer Erhöhung der Selektivität der Hydrierung führt, was auf die aufgrund des Massenwirkungsgesetzes zu erwartende Verminderung der Rückspaltung des Urethans in Alkohol und Isocyanat zurückzuführen ist, sondern auch die Aktivität des Katalysators und somit die Raum-Zeit-Ausbeute des gesamten Verfahrens erheblich steigert. Der Alkoholgehalt der Lösungsmittelmischung variiert von 0,1 bis 99,9 Gew.-%, bevorzugt von kleiner 50 Gew.-%, besonders bevorzugt von 5 bis 30 Gew.-%.

Zur kontinuierlichen Hydrierung wird ein Festbettreaktor bevorzugt. Der Festbettreaktor kann als Blasenreaktor betrieben werden, bevorzugt wird aber eine Rieselbettfahrweise. Vorzugsweise wird ein Rieselbettreaktor mit einem LHSV-Wert im Bereich von 0,1 bis 5 h⁻¹ (= L Reaktionslösung pro L Festbettkatalysator und Stunde). Gemäß einer besonders bevorzugten Ausführungsform des erfindungsgemäßen Verfahrens wird ein Rohrbündelreaktor verwendet und dieser in Rieselbettfahrweise betrieben.

### SPALTUNG:

Wie das Harnstoffverfahren führen die Mehrzahl aller beschriebenen Varianten zur Phosgen freien Isocyanatsynthese in der vorletzten Stufe zu Verbindungen, die zur Familie der Urethane gehören. Urethane, die auch als Carbamate bezeichnet werden, können als direkte Isocyanat-Vorläufer betrachtet werden. Schlüsselschritt ist in jedem Fall die Freisetzung des gewünschten Isocyanats, insbesondere durch thermische Spaltung des zugrunde liegenden Urethans bzw. Carbamats.

Analog beruht auch das erfindungsgemäße Verfahren im letzten Schritt auf der thermisch induzierten Spaltung von Urethanen. Dies kann in Anlehnung an grundsätzlich bekannte Verfahren zur Urethanspaltung erfolgen, d. h. in der Gas-oder Flüssigphase, mit oder ohne Lösemittel und mit oder ohne Katalysator (EP 0 126 299, EP 0 126 300, EP 0 355 443, EP 0 092 738, EP 0 396 977, EP 0 396 976). Vorzugsweise orientiert sich das erfindungsgemäße Verfahren in Bezug auf die Urethanspaltung im Wesentlichen an Erkenntnissen aus der Harnstofftechnologie gemäß EP 0 568 782. Im Detail vom Vorbild abweichende Verfahrensparameter (Druck, Temperatur, Katalysatormenge) ergeben sich aus der Tatsache, dass die Urethane des erfindungsgemäßen Verfahrens und der Harnstofftechnologie eine unterschiedliche Entstehungsgeschichte haben und sich aus diesem Grunde zwar nicht grundsätzlich aber doch im Spektrum der Nebenprodukte voneinander unterscheiden können.

In Anlehnung an den oben erwähnten Stand der Technik aus der Harnstofftechnologie erfolgt die Deblockierungsreaktion im Rahmen des erfindungsgemäßen Verfahrens bevorzugt dergestalt, dass das Urethan in flüssiger Phase ohne Verwendung von Lösemitteln in Gegenwart von 1 bis 2 000, bevorzugt 2 bis 1 000, besonders bevorzugt 5 bis 500 ppm eines geeigneten Katalysators in einer kombinierten Spalt- und Rektifizierkolonne bei einer Sumpftemperatur von 200 bis 300 °C, vorzugsweise von 215 bis 245 °C, und einem Sumpfdruck von 1 bis 50 mbar, vorzugsweise von 5 bis 30 mbar, thermisch gespalten und das gebildete Isocyanat als Roh-Isocyanat im Seitenabzug der Rektifizierkolonne wird, während der Alkohol am Kopf abgezogen wird. Die kombinierte Spalt- und Rektifizierkolonne, bestehend aus einem Unterteil für die Spaltung und einem Oberteil für die Rektifikation der Spaltprodukte, sollte mit hochwirksamen, vorzugsweise geordneten Packungen versehen und im Sumpf für dortige Energiezufuhr mit einem Fallfilmverdampfer ausgestattet sein. Um bei der Spaltung gebildete Nebenprodukte zu entfernen, wird aus dem Sumpf kontinuierlich Reaktionsgemisch in einer Menge von 2 bis 50 Gew.-%, vorzugsweise von 5 bis 25 Gew.-%, bezogen auf den Einsatz, entfernt. Die Dosierung des zu spaltenden Urethans erfolgt in das untere Drittel der Kolonne, kann alternativ aber auch in die Wälzung zum Fallfilmverdampfer geführt werden.

Aufgrund der Reaktivität der Isocyanatgruppe und der damit verbundenen Neigung zur Ausbildung unerwünschter höhermolekularer Nebenkomponenten ist es empfehlenswert, die mittlere Verweilzeit in der Spaltzone möglichst gering zu halten, was durch Minimierung des Flüssigkeitsvolumens durch entsprechende konstruktive Maßnahmen und durch Verwendung von geordneten Packungen mit geringem ,hold up' sowie durch möglichst zügige destillative Entfernung des gebildeten Isocyanats aus der Spaltzone erreicht wird.

Selbst bei Einhaltung bestmöglicher Bedingungen im Hinblick auf die mittlere Verweilzeit und die zügige Entfernung des gebildeten Isocyanats lässt sich die Bildung höhermolekularer Nebenprodukte nicht gänzlich unterdrücken, so dass zu deren Entfernung kontinuierlich anteilig Reaktionsmischung aus dem Sumpf ausgeschleust wird. Durch die Ausschleusung wird eine Verharzung des Materials weitgehend unterdrückt, so dass signifikante Störungen im Ablauf des technischen Prozesses, verursacht durch Belegungen und Verstopfungen der Apparatur, vermieden werden können.

Die Reinigung des aus der kombinierten Spalt- und Rektifizierkolonne abgezogenen Roh-Diisocyanats erfolgt durch Vakuumdestillation, wobei Vorlauf- und Destillationsrückstände in die kombinierte Spalt- und Rektifizierkolonne zurückgeführt werden können.

Geeignete Katalysatoren im Sinne des erfindungsgemäßen Verfahrens sind Halogenide oder Oxide von Metallen der Gruppen IB, IIB, IIIB, IVB, VB, VIB, VIIB, und VIIIB des Periodensystems. Vorzugsweise finden Chloride von Zink oder Zinn sowie Oxide von Zink, Mangan, Eisen oder Kobalt Verwendung.

Der Urethanspaltung vorgeschaltet ist eine grobe Vorreinigungsstufe, bei der zunächst die Lösemittel, z. B. Tetrahydrofuran (THF) und überschüssiges Butanol, durch Stickstoff-Strippung abgetrennt und im Anschluss mit Hilfe einer zweistufigen Kombination aus Kurzwegverdampfung und Dünnschichtverdampfung weitere niedrigsiedende Nebenkomponenten abgereichert, die im Zuge der Sequenz Urethanisierung/Hydrierung entstanden sind und/oder bereits als Verunreinigungen in den Einsatzstoffen vorgelegen haben. Gegebenenfalls kann auf die Strippung von Lösemittel (z. B. THF und Butanol) im Vorfeld der Verdampferkombination auch gänzlich verzichtet werden.

Dem gereinigten Urethan wird vor Einsatz in die Spaltung der Spaltkatalysator als ca. 5 % Lösung oder Suspension in dem Alkohol, der auch zur Herstellung des Urethans herangezogen wird, in einer Menge von 1 bis 2 000, bevorzugt von 2 bis 1000, besonders bevorzugt von 5 bis 500 ppm, bezogen auf das Volumen der Mischung im Spaltreaktor, zudosiert.

Die Erfindung wird durch die folgenden Beispiele näher erläutert.

### Beispiele

### I. URETHANISIERUNG:

### Beispiel:

157 g (0,63 Mol) MDI werden in 2,2 I wasserfreiem Tetrahydrofuran (THF) gelöst und portionsweise mit 343 g (4,63 Mol) n-Butanol versetzt. Die Lösung wird 5 Stunden unter Rühren auf Rückfluss geheizt (70 °C) und dann abgekühlt. Nachdem das Lösungsmittel (THF und überschüssiges Butanol) am Rotationsverdampfer abgezogen wurde, fielen 388 g (99 %) MDU als weißes Pulver an, mit einem Schmelzpunkt von 115 bis 117 °C und einem NCO-Gehalt von 0,8 %.

### II. HYDRIERUNG:

### A. Herstellung des Katalysators:

### Beispiel 1:

Ein Aluminiumoxid-Formkörper (Extrudat, d = 3 mm) mit einer BET-Oberfläche von ca. 33 m²/g und einer binodalen Porenverteilung mit einem Porenvolumen von 0,41 ml/g, wobei im Wesentlichen keine Poren mit einem Durchmesser von 2 bis 50 nm festgestellt werden, jedoch 100 % des Porenvolumens aus Makroporen mit einem Durchmesser im Bereich von 50 bis 10 000 nm bestand, wird mit einer wässrigen Ruthenium-(lll)-Nitrat-Lösung bei ca. 90 bis 100 °C beschichtet, indem die Katalysatorlösung auf das in Bewegung befindliche Trägermaterial aufgesprüht wird, wobei gleichzeitig Wasser verdampft. Die Katalysatorlösung weist eine Konzentration von 5 % Metall, bezogen auf das Gewicht der Lösung, auf. Der so beschichtete Träger wird bei einer Temperatur von 120 bis 180 °C erhitzt und anschließend mit einem Gemisch aus 50 % H₂ und 50 % N₂ bei 200 °C für 4 Stunden reduziert. Der so hergestellte Katalysator enthält 3 Gew.-% Ruthenium, bezogen auf das Gesamtgewicht des Katalysators. Die Eindringtiefe des Rutheniums beträgt 70 bis 90 µm. Das Verhältnis der Rutheniumoberfläche, bestimmt durch CO-Chemisorption, zur Oberfläche des unbeschichteten Trägermaterials, bestimmt nach BET, beträgt etwa 0,05. Der Aluminiumoxid-Formkörper besteht im Wesentlichen aus alpha- und gamma-Al₂O₃ (ca. 18 Gew.-%. SiO₂ und ca. 2 Gew.-% Alkali- und Erdalkalioxide Fe₂O₃ und TiO₂.

### Beispiel 2:

Ein Aluminiumoxid-Formkörper (Extrudat, d = 3 mm) ähnlicher Zusammensetzung wie der Träger des Beispiels 1 mit einer BET-Oberfläche von ca. 32 m²/g, trimodaler Porenverteilung und einem Porenvolumen nm 0,58 ml/g wird in analoger Weise wie in Beispiel 1 imprägniert. Das Porenvolumen des Trägermaterials resultiert aus 31 % Poren aus 2 bis 50 nm, 44 % Poren mit 50 bis 10 000 nm und 25 % Poren mit einem Porendurchmesser von größer 10 000 nm bis 5 µm. Der so hergestellte Katalysator enthält wie Beispiel 1 3 Gew.-% Ruthenium, und die Eindringtiefe beträgt 70 bis 90 µm.

### Beispiel 3:

Ein Aluminiumoxid-Formkörper (Extrudat d = 3 mm) mit einer Oberfläche von ca. 54 m²/g weist bei trimodaler Porenverteilung ein Porenvolumen von 0,77 ml/g auf. 40 % des Porenvolumens resultiert aus Poren mit einem Durchmesser von 2 bis 50 nm, 60 % aus Poren mit einem Porendurchmesser von 50 bis 10000 nm. Die Imprägnierung des Trägers, Kalzinierung und Reduktion des Katalysators erfolgen in gleicher Weise wie bei Beispiel 1. Der so hergestellte Katalysator enthält 3 Gew.-% Ruthenium, bezogen auf das Gesamtgewicht des Katalysators. Die Eindringtiefe beträgt 70 bis 90 nm. Der eingesetzte Aluminiumoxid-Formkörper umfasst die alpha-, theta- und gamma-Al₂O₃-Modifikationen.

### Beispiel 4:

Ein Aluminiumoxid-Formkörper Kugeln mit 2 bis 4 mm mit einer BET-Oberfläche von ca. 35 m²/g weist bei monomodaler Porenverteilung ein Porenvolumen von 0,5 ml/g auf. 42 % des Porenvolumens wird aus Mesoporen (2 bis 50 nm)und 58 % aus Makroporen (50 bis 10 000 nm) gebildet. Das Trägermaterial umfasst die theta- und gamma-Al₂O₃-Modifikationen. Die Imprägnierung, Kalzinierung und Reduktion erfolgen in gleicher Weise wie in Beispiel 1. Der so erhaltene trägergebundene Rutheniumkatalysator enthält 3 Gew.-% Ruthenium, bezogen auf das Gesamtgewicht des Katalysators. Die Eindringtiefe des Rutheniums beträgt 80 bis 120 µm.

### Vergleichsbeispiel 1:

Ein Titandioxid-Formkörper (Extrudat, d = 2 mm) im Wesentlichen bestehend aus einem Gemisch aus Rutil und Anatas mit einer BET-Oberfläche von 45 m²/g weist bei monomodaler Porenverteilung ein Porenvolumen von 0,35 ml/g auf. Das Porenvolumen wird zu 100 % aus Mesoporen (2 bis 50 nm) gebildet. Der Formkörper wird in analoger Weise wie in Beispiel 1 imprägniert, die Trocknung erfolgt jedoch bei 150 bis 160 °C, und die anschließende Reduktion erfolgt bei 180 °C innerhalb von 4 Stunden. Der so hergestellte Katalysator enthält 3 Gew.-% Ruthenium, bezogen auf das Gesamtgewicht des Katalysators. Die Eindringtiefe beträgt 90 bis 120 µm.

### Vergleichsbeispiel 2:

Ein Aluminiumoxid-Formkörper (Extrudat d = 1,2 nm) aus im Wesentlichen gamma-Al₂O₃ mit einer BET-Oberfläche von 220 m²/g hat ein Porenvolumen von 0,65 ml/g, wobei 95 % des Porenvolumens aus Mesoporen (2 bis 50 nm) und 5 % des Porenvolumens aus Makroporen (50 bis 10 000 nm) gebildet werden. Der Träger wird mit einer wässrigen Ruthenium-(III)-nitrat-Lösung bei Raumtemperatur imprägniert. Die Katalysatorlösung besitzt eine Konzentration von 5 % Metall, bezogen auf das Gewicht der Lösung. Der imprägnierte Träger wird bei einer Temperatur von 150 bis 160 °C erhitzt und anschließend mit einem Gemisch aus 50 % H₂ und 50 % N₂ bei 180 °C für 4 Stunden reduziert. Der so hergestellte Katalysator enthält 5 Gew.-% Ruthenium, bezogen auf das Gesamtgewicht des Katalysators. Die Eindringtiefe beträgt bis 600 µm.

### B. Durchführung der Hydrierreaktion

### Beispiel 1:

### Herstellung einer MDU-Lösung mit 10 Gew.-% MDU, 10 Gew.-% n-Butanol und 80 Gew.-% THF

In einer 5-L-Dreihalskolbenrührappartur mit beheizbarem Tropftrichter werden 2 400 g THF (33,3 mol) und 300 g n-BuOH (4,05 mol) vorgelegt. Die Lösung wird bis zum Sieden (ca. 70 °C) erhitzt, danach werden zügig 188,4 g MDI (0,75 mol) als Schmelze zugetropft. Die Mischung wird bis zur Vervollständigung der Reaktion unter Rückfluss gehalten (ca. 6 Stunden). Die Vollständigkeit der Reaktion wird mittels NCO-Zahlbestimmung und IR-Spektroskopie überprüft.

Wenn ein anderer MDU- und/oder n-Butanolgehalt gewünscht wird, werden die Eduktmengen entsprechend angepasst.

### Beispiel 2:

### Hydrierung von MDU-Lösungen mit unterschiedlichen n-Butanolgehalten im Autoklaven bei 100°C

Dieses Beispiel soll den Einfluss des n-Butanols auf die Katalysatoraktivität verdeutlichen.

In Anlehnung an Beispiel 1 werden drei 10 Gew.-%ige MDU-Lösungen mit unterschiedlichen n-Butanolgehalten (0, 10 und 20 Gew.-% in der Endlösung, MDI der Fa. Aldrich) hergestellt. Diese Lösungen werden in einem 1-L-Laborautoklaven mit Katalysatorkorb bei 100° C und 80 bar hydriert. Es werden jeweils 600 g MDU-Lösung und 48,3 g erfindungsgemäßer Katalysator eingesetzt. Nach 5 Stunden wird dem Reaktor eine Probe entnommen, die mittels HPLC/CLND, HPLC/MS und GC-KAS-MS analysiert wird. Das Ergebnis ist in Tabelle 1 dargestellt. Es ist deutlich zu erkennen, dass mit zunehmendem n-Butanolgehalt der Reaktionsmischung nach 5 Stunden weniger hydrierbare Zwischenprodukte im Endprodukt enthalten sind und der H₁₂MDU-Gehalt höher ist, gleichbedeutend mit einem deutlichen Anstieg der Reaktionsgeschwindigkeit. Der Anteil des 4,4'-trans-trans-Isomeren ist mit ca. 8 % erfindungsgemäß niedrig.

**Tabelle 1**

| Ergebnis der Hydrierung von MDU-Lösung mit unterschiedlichen n-Butanolgehalten. | | | |
|---|---|---|---|
| Hydrierbedingungen 100°C, 80 bar. Alle Angaben in Gew.-%. | | | |
| **Reaktionsmischung Nr.** | **1** | **2** | **3** |
| **Eduktzusammensetzung** | | | |
| MDU | 10 | 10 | 10 |
| THF | 90 | 80 | 70 |
| n- Butanol | 0 | 10 | 20 |

| **Produktzusammensetzung**^{**1)**} | | | |
|---|---|---|---|
| MDU | 6,8 | 0 | 0 |
| Hydrierbare Zwischenprodukte²⁾ | 54,9 | 15 | 3,3 |
| H₁₂MDU | 36,4 | 81,9 | 92,3 |
| Nebenprodukte | 1,9 | 3,1 | 4,4 |
| 4,4'-Trans-trans-Anteil ³⁾ | 7 | 8 | 8 |

| | | | |
|---|---|---|---|
| ¹⁾ n-Butanol und THF rausgerechnet ²⁾ Nur solche, die im weiteren Verlauf der Reaktion zu H₁₂MDU weiterhydriert werden können. ³⁾ Anteil des trans-trans-H₁₂MDU bezogen auf die Summe der Anteile aller H₁₂MDU-Isomeren. | | | |

### Beispiel 3:

### Hydrierung von MDU-Lösungen mit unterschiedlichen n-Butanolgehalten im Autoklaven bei 120 °C

Dieses Beispiel soll den positiven Einfluss des n-Butanols auf die Selektivität verdeutlichen.

In Anlehnung an Beispiel 1 werden drei 10 Gew.-%ige MDU-Lösungen mit unterschiedlichen n-Butanolgehalten (0, 10 und 20 Gew.-% in der Endlösung, MDI der Fa. Aldrich) hergestellt. Diese Lösungen werden in einem 1-L-Laborautoklaven mit Katalysatorkorb bei 120 °C und 80 bar hydriert. Es werden jeweils 600 g MDU-Lösung und 48,3 g erfindungsgemäßer Katalysator eingesetzt. Nach 4 Stunden wurde dem Reaktor eine Probe entnommen, die mittels HPLC/CLND, HPLC/MS und GC-KAS-MS analysiert wird. Das Ergebnis ist in Tabelle 2 dargestellt. Bei allen Versuchen ist nach 4 Stunden kein hydrierbares Zwischenprodukt mehr detektierbar. Es ist deutlich zu erkennen, dass mit zunehmendem n-Butanolgehalt der Reaktionsmischung der Nebenproduktanteil sinkt, gleichbedeutend mit einem deutlichen Anstieg der Selektivität. Der Anteil des 4,4'-trans-trans-Isomeren ist mit ca. 8 % erfindungsgemäß niedrig.

**Tabelle 2**

| Ergebnis der Hydrierung von MDU-Lösung mit unterschiedlichen n-Butanolgehalten. | | | |
|---|---|---|---|
| Hydrierbedingungen: 120°C, 80 bar. Alle Angaben in Gew.-%. | | | |
| **Reaktionsmischung Nr.** | **1** | **2** | **3** |
| **Eduktzusammensetzung** | | | |
| MDU | 10 | 10 | 10 |
| THF | 90 | 80 | 70 |
| n- Butanol | 0 | 10 | 20 |

| **Produktzusammensetzung**^{**1)**} | | | |
|---|---|---|---|
| MDU | 0 | 0 | 0 |
| Hydrierbare Zwischenprodukte²⁾ | 0 | 0 | 0 |
| H₁₂MDU | 92 | 93,5 | 94,8 |
| Nebenprodukte | 8 | 6,5 | 5,2 |
| 4,4'-Trans-trans-Anteil ³⁾ | 8 | 7 | 7 |

| | | | |
|---|---|---|---|
| ¹⁾ n-Butanol und THF rausgerechnet ²⁾ Nur solche, die im weiteren Verlauf der Reaktion zu H₁₂MDU weiterhydriert werden können. ³⁾ Anteil des trans-trans-H₁₂MDU bezogen auf die Summe der Anteile aller H₁₂MDU-Isomeren. | | | |

### Beispiel 4:

### Hydrierung von MDU-Lösungen mit unterschiedlichen n-Butanolgehalten im Rieselbettreaktor bei 100 °C

In Anlehnung an Beispiel 1 werden drei 10 Gew.-%ige MDU-Lösungen mit unterschiedlichen n-Butanolgehalten (0, 5 und 10 Gew.-% in der Endlösung) hergestellt. Diese Lösungen werden in einem Rieselbettreaktor, gefüllt mit 14,5 g erfindungsgemäßem Katalysator, bei 100 °C und 80 bar hydriert. Die entnommenen Proben werden mittels HPLC/CLND, HPLC/MS und GC-KAS-MS analysiert. Das Ergebnis ist in Tabelle 3 dargestellt.

Es ist deutlich zu erkennen, dass mit zunehmendem n-Butanolgehalt der Reaktionsmischung der Nebenproduktanteil sinkt, gleichbedeutend mit einem deutlichen Anstieg der Selektivität. Gleichzeitig verringert sich der nicht umgesetzte MDU-Anteil. Der Anteil des 4,4'-trans-trans-Isomeren ist mit ca. 8,9 bis 9,8 % erfindungsgemäß niedrig.

**Tabelle 3**

| Ergebnis der kontinuierlichen Hydrierung von MDU-Lösung mit unterschiedlichen n- | | | |
|---|---|---|---|
| Butanolgehalten. Hydrierbedingungen: 100 °C, 80 bar. Alle Angaben in Gew.-%. | | | |
| **Reaktionsmischung Nr.** | **1** | **2** | **3** |
| **Eduktzusammensetzung** | | | |
| MDU | 10 | 15 | 10 |
| THF | 90 | 80 | 80 |
| n- Butanol | 0 | 5 | 10 |

| **Produktzusammensetzung**^{**1)**} | | | |
|---|---|---|---|
| MDU | 8,6 | 0 | 0 |
| Hydrierbare Zwischenprodukte²⁾ | 47,6 | 0,4 | 0 |
| H₁₂MDU | 39,1 | 95,7 | 98,1 |
| Nebenprodukte | 4,6 | 3,9 | 2,0 |
| 4,4'-Trans-trans-Anteil ³⁾ | 9,6 | 8,9 | 9,8 |

| | | | |
|---|---|---|---|
| ¹⁾ n-Butanol und THF rausgerechnet ²⁾ Nur solche, die im weiteren Verlauf der Reaktion zu H₁₂MDU weiterhydriert werden können. ³⁾ Anteil des trans-trans-H₁₂MDU bezogen auf die Summe der Anteile aller H₁₂MDU-Isomeren. | | | |

### III. SPALTUNG:

### A. Aufarbeitung einer H₁₂MDU-haltigen THF-Butanol-Lösung aus der Hydrierung

Die aus der Hydrierung stammende THF-Butanol-Lösung enthält, je nach Wirkungsgrad der vorgeschalteten Flash-Stufe, ~20% H₁₂MDU und ~ 80% Lösemittelanteil.

Um Peroxidbildung bei der Aufarbeitung zu vermeiden, wird diese Lösung kontinuierlich in einer zweistufigen Kurzweg-/Dünnschichtverdampferanlage aufgearbeitet. Aus sicherheitstechnischen Gründen empfiehlt sich die Einhaltung kurzer Kontaktzeiten zwischen Produkt und Heizfläche.

### Beispiel:

2 500 g/h der H₁₂MDU-haltigen THF-Butanol-Lösung werden kontinuierlich auf einen Kurzwegverdampfer gefahren, der bei 140 °C und 700 mbar betrieben wird. Das anfallende Destillat wird bei -5 °C kondensiert Über einen Zeitraum von 7 Stunden fallen 13 903 g Destillat an. Das Destillat ist H₁₂MDU-frei und besteht zu 81 % aus THF und 19 % Butanol.

Der Ablauf des Kurzwegverdampfers wird auf einen Dünnschichtverdampfer mit aufgesetzter Kolonne gefahren, um das restliche THF-Butanol-Gemisch, sowie leichtsiedende Verunreinigungen abzutrennen. Die aufgesetzte Kolonne hat die Aufgabe, mitgerissenes H₁₂MDU zurückzuhalten.

Die Destillation am Dünnschichtverdampfer erfolgt bei 230 °C und 6 mbar. Die aufgesetzte Kolonne ist mit 170 °C begleitbeheizt. Die Kondensation des Kopfprodukts erfolgt ebenfalls bei -5 °C. In dem beschriebenen Zeitraum fallen 246 g Kopfprodukt und 3 220 g H₁₂MDU an. Das Kopfprodukt besteht zu 70 % aus Butanol-THF, sowie zu 30 % aus Vorlaufkomponenten.

Das H₁₂MDU enthält <0,05 % THF und <0,5 % Butanol. Es kann ohne weitere destillative Reinigung direkt in die Spaltung eingesetzt werden.

### B. Thermische Spaltung von H₁₂MDU in H₁₂MDI und Butanol

Die Spaltung erfolgt in einer kombinierte Spalt- und Rektifizierkolonne bei 230 °C und 10 mbar, in Gegenwart von etwa 10 bis 20 ppm Zinn-2-chlorid als Katalysator.

Das gebildete Diisocyanat wird als Roh-Diisocyanat im Seitenabzug gewonnen, während der reine Alkohol am Kopf abgezogen wird. Die kombinierte Spalt- und Rektifizierkolonne ist für die Energiezufuhr mit einem Fallfilmverdampfer ausgestattet. In die Wälzung des Fallfilmverdampfers erfolgt die Einspeisung des H₁₂MDU und des Katalysators.

### Beispiel:

750 g H₁₂MDU-Schmelze (140 °C) werden pro Stunde in die Wälzung des Fallfilmverdampfers der Spalt- und Rektifizierkolonne gefahren. Die Spaltgase H₁₂MDI und Butanol werden in zwei hintereinander geschalteten Kondensatoren bei 85 °C und -25 °C auskondensiert. Das gewonnene, etwa 97-%ige Roh-H₁₂MDI wird einer Reindestillation zugeführt, wobei 400 g/h H₁₂MDI mit einer Reinheit von > 99,5 % erhalten werden. 260 g/h Butanol fallen als Kopfprodukt der Spalt- und Rektifizierkolonne an. Dieses Butanol hat eine Reinheit von > 99,5 % und ist mit geringen Anteilen von Monoisocyanato-monourethan verunreinigt. Zur Aufrechterhaltung der Massenkonstanz innerhalb der Spalt- und Rektifizierkolonne und Vermeidung von Belegungen und Verstopfungen der Spaltapparatur werden 60 g/h aus dem Wälzkreislauf ausgeschleust.

## Patentansprüche

1. Verfahren zur Herstellung von cycloaliphatischen Isocyanaten aus den korrespondierenden aromatischen Isocyanaten, welche einen oder mehrere aromatische Ringe und eine oder mehrere, direkt und/oder indirekt an einem oder verschiedenen aromatischen Ringen gebundene Isocyanatgruppen aufweisen, umfassend im Wesentlichen die drei folgenden Stufen:
1. Urethanisierung des aromatischen Isocyanats,
2. Hydrierung des aromatischen Urethans mit Wasserstoff in Gegenwart eines geträgerten Katalysators, der als Aktivmetall Ruthenium alleine oder zusammen mit mindestens einem Metall der I., VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, und wobei der Katalysatorträger eine BET-Oberfläche im Bereich von größer 30 m²/g bis kleiner 70 m²/g aufweist und mehr als 50 % des Porenvolumens des Katalysatorträgers Makroporen mit einem Porendurchmesser von größer 50 nm und weniger als 50 % Mesoporen mit einem Porendurchmesser von 2 bis 50 nm sind,
3. Spaltung des hydrierten Urethans zum aliphatischen Isocyanat,
wobei aromatische Isocyanate, ausgewählt aus
Bis[4-isocyanatophenyl]methan, 2-Isocyanatophenyl-4'-isocyanatophenylmethan, 2-Isocyanatophenyl-2'-isocyanatophenylmethan (MDI) und mehrkernige methylenverbrückte Isocyanatophenyle (PMDI) sowie Gemische davon, 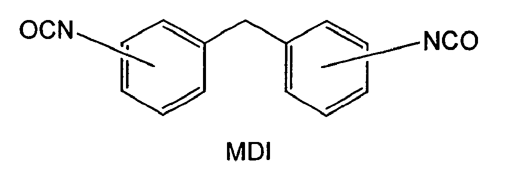 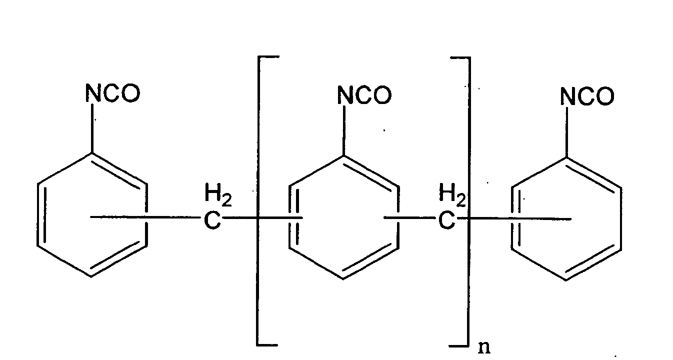 PMDI, n = 1 - 10
4,4'-Diisocyanato-3,3'-dimethylphenylmethan, 2,4'-Diisocyanato-3,3'-dimethylphenylmethan, 2,2'-Diisocyanato-3,3'-dimethylphenylmethan sowie Gemische davon, 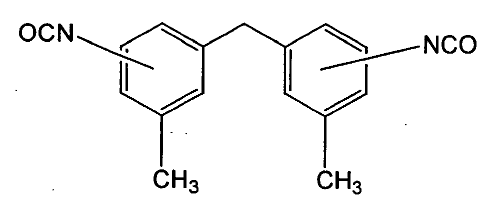 1,2-Diisocyanatobenzol, 1,3-Diisocyanatobenzol und 1,4-Diisocyanatobenzol sowie Gemische davon, 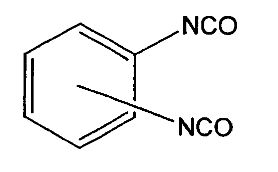 2,4-Diisocyanatotoluol, 2,6-Diisocyanatotoluol sowie deren Mischungen, 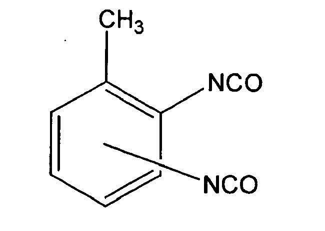 1,6-Diisocyanatonaphthalin, 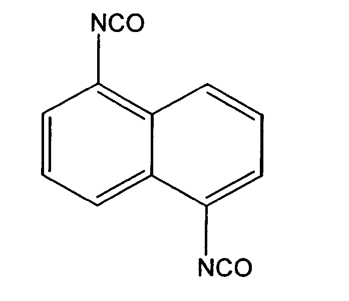 MXDI und TMXDI , 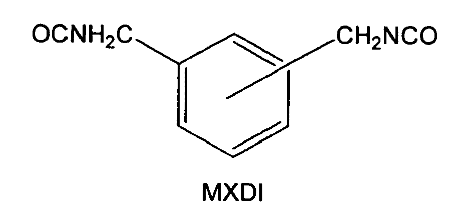 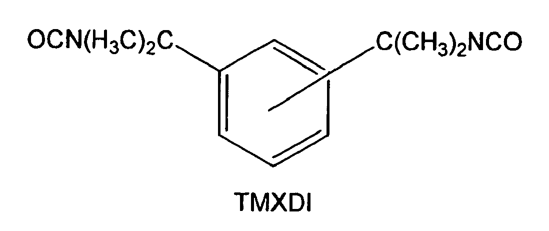 eingesetzt werden.

2. Verfahren nach Anspruch 1,
**dadurch gekennzeichnet,**
**dass** die Urethanisierung in der 1. Stufe kontinuierlich oder diskontinuierlich, in An- oder Abwesenheit eines Lösemittels oder Lösemittelgemischs, in An- oder Abwesenheit eines Katalysators, bei Temperaturen von 20 bis 160 °C durchgeführt wird.

3. Verfahren nach mindestens einem der Ansprüche1 bis 2,
**dadurch gekennzeichnet,**
**dass** die Urethanisierung in einem Alkohol, insbesondere in n-Butanol, durchgeführt wird.

4. Verfahren nach mindestens einem der Ansprüche 1 bis 3,
**dadurch gekennzeichnet,**
**dass** das auf den Katalysator der 2. Stufe aufgebrachte Aktivmetall eine Eindringtiefe in den Träger im Bereich von 20 bis 500 µm, insbesondere 25 bis 250 µm, aufweist.

5. Verfahren nach mindestens einem der Ansprüche 1 bis 4,
**dadurch gekennzeichnet,**
**dass** das Verhältnis der Oberflächen des Aktivmetalls, bestimmt durch CO-Pulschemisorption, und des Katalysatorträgers, bestimmt nach BET, größer als 0,01, insbesondere 0,03 bis 0,3, ist.

6. Verfahren nach mindestens einem der Ansprüche 1 bis 5,
**dadurch gekennzeichnet,**
**dass** das Trägermaterial ausgewählt ist aus der Reihe der kristallinen und amorphen Oxide und Silikate, insbesondere ausgewählt aus der Reihe Al₂O₃, SiO₂, TiO₂, ZrO₂, MgO, ZnO und Alumosilikate.

7. Verfahren nach mindestens einem der Ansprüche 1 bis 6, **dadurch gekennzeichnet,**
**dass** der Katalysatorträger eine BET-Oberfläche im Bereich von 32 bis 67 m²/g aufweist, die Eindringtiefe der Aktivmetalle im Bereich von 50 bis 200 µm und die Ru-Menge im Bereich von 0,2 bis 3 Gew.-%, bezogen auf den Katalysator, liegt und mindestens 55 % des Porenvolumens des Katalysatorträgers aus Makroporen und weniger als 45 % aus Mesoporen gebildet wird.

8. Verfahren nach mindestens einem der Ansprüche 1 bis 7,
**dadurch gekennzeichnet,**
**dass** man die Hydrierung in einem kontinuierlich oder diskontinuierlich zu betreibenden Suspensions- oder Festbett-Hydrierreaktor bei einer Temperatur im Bereich von 20 bis 250 °C, insbesondere von 50 bis 150 °C, besonders bevorzugt 70 bis 120°C und einem Wasserstoffpartialdruck im Bereich von 1 bis 30 MPa, insbesondere von 3 bis 15 MPa, durchführt.

9. Verfahren nach mindestens einem der Ansprüche 1 bis 8,
**dadurch gekennzeichnet,**
**dass** man die Hydrierung in einem Festbettreaktor, insbesondere in einem Rohrbündelreaktor, in Rieselbettfahrweise durchführt.

10. Verfahren nach mindestens einem der Ansprüche 1 bis 9,
**dadurch gekennzeichnet,**
**dass** man einen geträgerten Katalysator verwendet, dessen Aktivmetall Ruthenium auf einen Träger aufgebracht wurde durch Besprühen des Trägers mit einer verdünnten Rutheniumsalzlösung, insbesondere einer Rutheniumnitrosylnitratlösung, bei einer Temperatur von mindestens 80 °C mit anschließender Temperung und Aktivierung des Katalysators durch Reduktion in einem wasserstoffhaltigen Gas.

11. Verfahren nach mindestens einem der Ansprüche 1 bis 10,
**dadurch gekennzeichnet,**
**dass** Verbindungen, ausgewählt aus Dialkyl 4,4'-methylendicarbanilat, Dialkyl 2,4'-methylendicarbanilat, Dialkyl 2,2'-methylendicarbanilat und mehrkernige methylenverbrückte Alkyl-Carbanilate (PMDU) sowie Gemische davon, 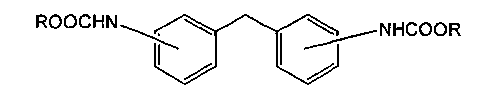 R = C₁-C₆-Alkyl, bevorzugt n-Butyl, 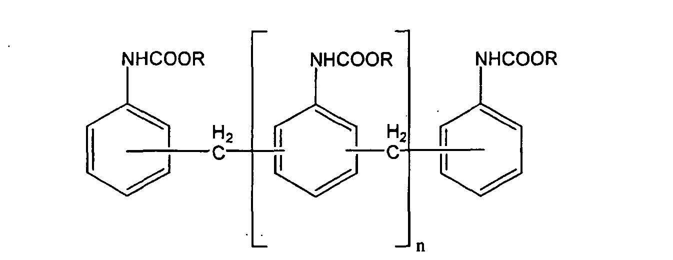 PMDU, R = C₁-C₆-Alkyl, bevorzugt n-Butyl, [n = 1 - 10]
Dialkyl 4,4'-methylen-3,3'-dicarbanilat, Dialkyl 2,4'-methylen-3,3'-dicarbanilat, Dialkyl 2,2'-methylen-3,3'-dicarbanilat sowie Gemische davon, 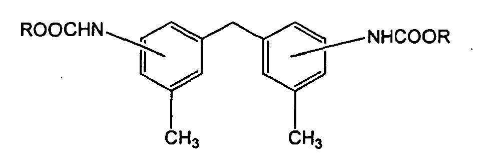 R = C₁-C₆-Alkyl, bevorzugt n-Butyl
Dialkyl 1,2-Phenyldicarbamat, Dialkyl 1,3-Phenyldicarbamat und Dialkyl 1,4-Phenyldicarbamat sowie Gemische davon, 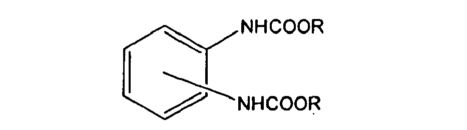 R = C₁-C₆-Alkyl, bevorzugt n-Butyl
Dialkyl 2,4-Toluoldicarbamat, Dialkyl 2,6-Toluoldicarbamat sowie deren Mischungen, 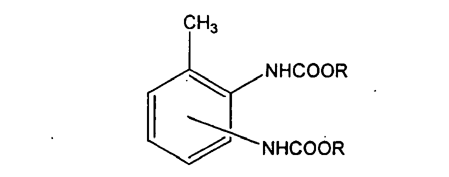 R = C₁-C₆-Alkyl, bevorzugt n-Butyl
Dialkyl 1,6-Naphthalindicarbamat, 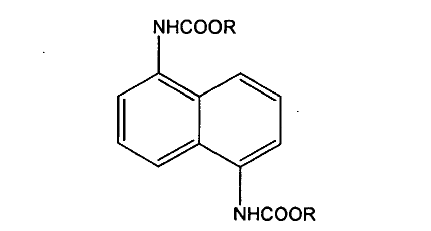 R = C₁-C₆-Alkyl, bevorzugt n-Butyl
die zum so genannten MXDI und TMXDI korrespondierenden Urethane, 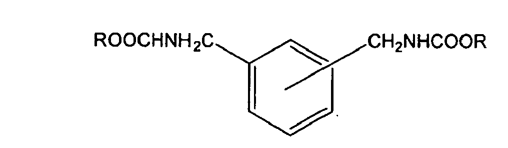 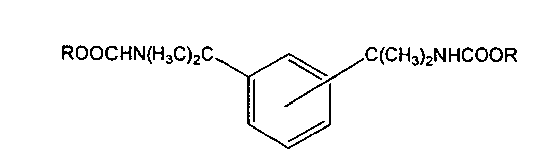 (R = Alkyl, bevorzugt n-Butyl)
hydriert werden.

12. Verfahren nach mindestens einem der Ansprüche 1 bis 11,
**dadurch gekennzeichnet,**
**dass** man ein Dialkyl 4,4'-(C₁ bis C₄)alkan-dicarbanilat und/oder ein 2,4'-und/oder 2,2'-Isomeres oder Gemische hiervon, hydriert.

13. Verfahren nach Anspruch 12,
**dadurch gekennzeichnet,**
**dass** man ein Dibutyl 4,4'-methylendicarbanilat oder ein Isomeres oder ein Gemisch hydriert.

14. Verfahren nach mindestens einem der Ansprüche 1 bis 13,
**dadurch gekennzeichnet,**
**dass** aus verbrückten zweikernigen Ausgangsprodukten Hydrierprodukte mit einem trans-trans-Isomerenanteil von < 30 %, bevorzugt von < 20%, ganz besonders bevorzugt von 5 bis 15 %, hergestellt werden.

15. Verfahren nach mindestens einem der Ansprüche 1 bis 14 zur Hydrierung von Dibutyl 4,4'-methylendicarbanilat zu Dibutyl 4,4'-methylendicyclohexylcarbamat mit einem trans-trans-Isomerenanteil von < 30 %, bevorzugt von < 20 %, besonders bevorzugt von 5 bis 15 %.

16. Verfahren nach mindestens einem der Ansprüche 1 bis 15,
**dadurch gekennzeichnet,**
**dass** die Hydrierung in einem Lösemittel oder Lösemittelgemisch, insbesondere aus Alkoholen und/oder Ethern, durchgeführt wird.

17. Verfahren nach Anspruch 16,
**dadurch gekennzeichnet,**
**dass** der Alkohol dem Alkoholrest des Urethans entspricht.

18. Verfahren nach den Ansprüchen 16 bis 17,
**dadurch gekennzeichnet,**
**dass** n-Butanol und Tetrahydrofuran eingesetzt werden.

19. Verfahren nach mindestens einem der Ansprüche 1 bis 18,
**dadurch gekennzeichnet,**
**dass** die Spaltung der hydrierten Urethane in der 3. Stufe in der Gas- oder Flüssigphase, mit oder ohne Katalysator, in An- oder Abwesenheit von Lösemitteln, kontinuierlich oder diskontinuierlich, erfolgt.

20. Verfahren nach mindestens einem der Ansprüche1 bis 19,
**dadurch gekennzeichnet,**
**dass** die Spaltung in einer kombinierten Spalt- und Rektifizierkolonne erfolgt.

21. Verfahren nach mindestens einem der Ansprüche 1 bis 20,
**dadurch gekennzeichnet,**
**dass** die Spaltung in flüssiger Phase ohne zusätzliche Lösemittel erfolgt.

22. Verfahren nach mindestens einem der Ansprüche 1 bis 21,
**dadurch gekennzeichnet,**
**dass** die Spaltung in Gegenwart mindestens eines Katalysators erfolgt.

23. Verfahren nach Anspruch 22,
**dadurch gekennzeichnet,**
**dass** 1 bis 2 000 ppm Katalysator, bezogen auf das Volumen der Mischung im Spaltreaktor, eingesetzt werden.

24. Verfahren nach mindestens einem der Ansprüche 1 bis 23,
**dadurch gekennzeichnet,**
**dass** die höhermolekularen Nebenprodukte aus dem Sumpf der Spaltkolonne ausgeschleust werden.

25. Verfahren nach mindestens einem der Ansprüche 1 bis 24,
**dadurch gekennzeichnet,**
**dass** die Reinigung des aus der kombinierten Spalt- und Rektifizierkolonne abgezogenen Roh-Isocyanats durch Vakuumdestillation, wobei Vorlauf und Destillationsrückstände in die kombinierte Spalt- und Rektifizierkolonne zurückgeführt werden können, erfolgt.

26. Verfahren nach mindestens einem der Ansprüche 1 bis 25,
**dadurch gekennzeichnet,**
**dass** die Spaltung thermisch bei einer Temperatur von 200 bis 300 °C erfolgt.

27. Verfahren nach mindestens einem der Ansprüche 1 bis 26,
**dadurch gekennzeichnet,**
**dass** der Urethanspaltung der 3. Stufe eine grobe Vorreinigungsstufe vorgeschaltet ist, bei der zunächst Lösemittel durch Stickstoff-Strippung abgetrennt und im Anschluss mit Hilfe einer zweistufigen Kombination aus Kurzwegverdampfung und Dünnschichtverdampfung weitere niedrigsiedende Nebenkomponenten abgereichert werden.

28. Verfahren nach mindestens einem der Ansprüche 1 bis 27,
**dadurch gekennzeichnet,**
**dass** das gesamte Verfahren vollständig kontinuierlich, teilweise kontinuierlich oder diskontinuierlich durchgeführt wird.

29. Verfahren nach mindestens einem der Ansprüche 1 bis 28 zur Herstellung von H₁₂MDI mit einem trans-trans-Isomerenanteil von < 30 %, bevorzugt von < 20 %, besonders bevorzugt von 5 bis 15 %, aus MDI, umfassend im Wesentlichen die drei folgenden Stufen:
1. Urethanisierung des MDI,
2. Hydrierung des MDU mit Wasserstoff in Gegenwart eines geträgerten Katalysators, der als Aktivmetall Ruthenium alleine oder zusammen mit mindestens einem Metall der I., VII. oder VIII. Nebengruppe des Periodensystems in einer Menge von 0,01 bis 20 Gew.-% Aktivmetalle, bezogen auf den geträgerten Katalysator, auf einen Träger aufgebracht enthält, und wobei der Katalysatorträger eine BET-Oberfläche im Bereich von größer 30 m²/g bis kleiner 70 m²/g aufweist und mehr als 50 % des Porenvolumens des Katalysatorträgers Makroporen mit einem Porendurchmesser von größer 50 nm und weniger als 50 % Mesoporen mit einem Porendurchmesser von 2 bis 50 nm sind,
3. Spaltung des hydrierten Urethans zum H₁₂MDI.

30. Verfahren nach mindestens einem der vorherigen Ansprüche,
**dadurch gekennzeichnet,**
**dass** ein schwefel- und/oder phosphor- und/oder chlorfreies Startmaterial eingesetzt wird.

## Claims

1. A process for preparing a cycloaliphatic isocyanate from a corresponding aromatic isocyanate which has one or more aromatic rings and one or more isocyanate groups bound directly and/or indirectly to an aromatic ring or various aromatic rings, comprising essentially the following three steps:
1. Urethanization of the aromatic isocyanate,
2. Hydrogenation of the aromatic urethane by means of hydrogen in the presence of a supported catalyst containing ruthenium alone or ruthenium together with at least one metal of transition group I, VII or VIII of the Periodic Table as active metal in an amount of from 0.01 to 20% by weight of total active metals, based on the supported catalyst, applied to a support, where the catalyst support has a BET surface area in the range of from > 30 m²/g to < 70 m²/g and more than 50% of the pore volume of the catalyst support is made up by macropores having a pore diameter greater than 50 nm and less than 50% of the pore volume is made up by mesopores having a pore diameter of from 2 to 50 nm,
3. Dissociation of the hydrogenated urethane to give the aliphatic isocyanate,
an aromatic isocyanate selected from among
bis[4-isocyanatophenyl]methane, 2-isocyanatophenyl-4'-isocyanatophenylmethane, 2-isocyanatophenyl-2'-isocyanatophenylmethane (MDI) and a multi- ring methylene-bridged isocyanatophenyl (PMDI) and mixtures thereof, 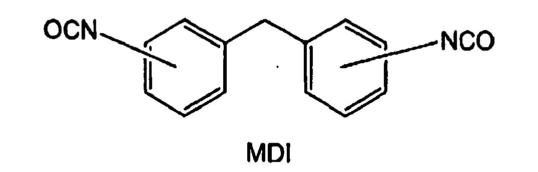 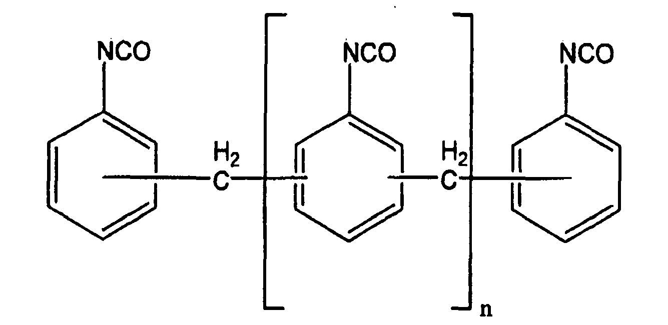 PMDI, n = 1 - 10
4,4'-diisocyanato-3,3'- dimethylphenylmethane, 2,4'-diisocyanato-3,3'-dimethylphenylmethane, 2,2'-diisocyanato-3,3'- dimethylphenylmethane and mixtures thereof, 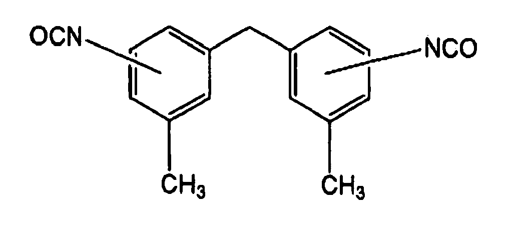 1,2-diisocyanatobenzene, 1,3-diisocyanatobenzene and 1,4-diisocyanatobenzene and mixtures thereof, 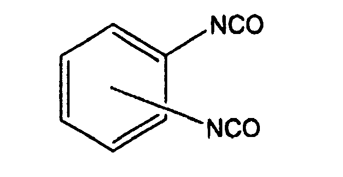 2,4-diisocyanatotoluene, 2,6-diisocyanatotoluene and mixtures thereof, 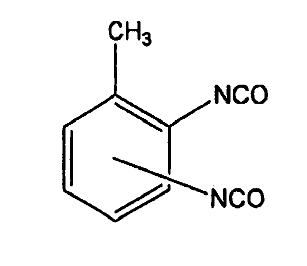 1,6-diisocyanatonaphthalene, 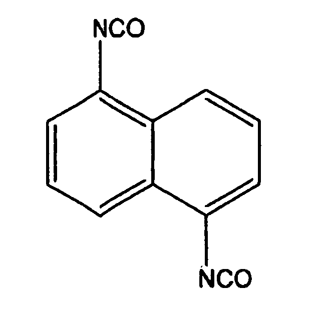 MXDI and TMXDI, 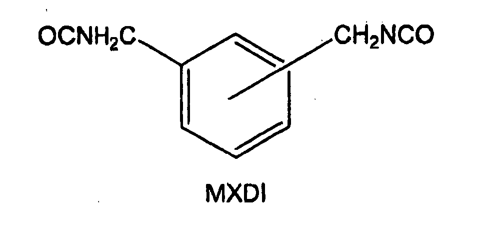 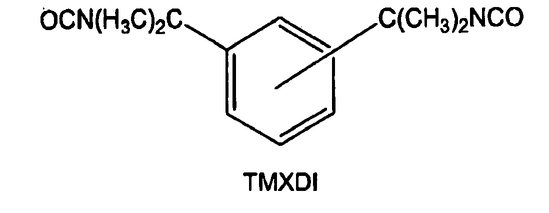 being used.

2. A process according to claims 1,
**characterized in that**
the urethanization in the 1st step is carried out continuously or batchwise, in the presence or absence of a solvent or solvent mixture, in the presence or absence of a catalyst, at a temperature of from 20 to 160°C.

3. A process according to at least one of claims 1 to 2,
**characterized in that**,
the urethanization is carried out in an alcohol, in particular in n-butanol.

4. A process according to at least one of claims 1 to 3,
**characterized in that**
the active metal applied to the catalyst of the 2nd step has a penetration depth into the support in the range from 20 to 500 µm, in particular from 25 to 250 µm.

5. A process according to at least one of claims 1 to 4,
**characterized in that**
the ratio of the surface area of the active metal, determined by CO pulse chemisorption, to that of the catalyst support, determined by BET, is greater than 0.01, in particular from 0.03 to 0.3.

6. A process according to at least one of claims 1 to 5,
**characterized in that**
the support material is selected from the group consisting of crystalline and amorphous oxides and silicates, in particular the group consisting of Al₂O₃, SiO₂, TiO₂, ZrO₂, MgO, ZnO and aluminosilicates.

7. A process according to at least one of claims 1 to 6,
**characterized in that**
the catalyst support has a BET surface area in the range from 32 to 67 m²/g, the penetration depth of the active metal is in the range from 50 to 200 µm, the amount of Ru is in the range from 0.2-3% by weight, based on the catalyst, and at least 55% of the pore volume of the catalyst support is made up by macropores and less than 45% is made up by mesopores.

8. A process according to at least one of claims 1 to 7,
**characterized in that**
the hydrogenation is carried out in a continuously or discontinuously operated suspension or fixed-bed hydrogenation reactor at a temperature in the range from 20 to 250°C, in particular from 50 to 150°C, particularly preferably from 70 to 120°C and a hydrogen partial pressure in the range from 1 to 30 MPa, in particular from 3 to 15 MPa.

9. A process according to at least one of claims 1 to 8,
**characterized in that**
the hydrogenation is carried out in a fixed-bed reactor, in particular in a shell-and-tube reactor, in the trickle bed mode.

10. A process according to at least one of claims 1 to 9,
**characterized in that**
a supported catalyst whose active metal ruthenium has been applied to a support by spraying the support with a dilute ruthenium salt solution, in particular a ruthenium nitrosyl nitrate solution, at a temperature of at least 80°C with subsequent heat treatment and activation of the catalyst by reduction in a hydrogen-containing gas is used.

11. A process according to at least one of claims 1 to 10,
**characterized in that**
a compound selected from among dialkyl 4,4'-methylenedicarbanilate, dialkyl 2,4'-methylenedicarbanilate, dialkyl 2,2'-methylenedicarbanilate and multi-ring methylene-bridged alkyl carbanilates (PMDU) and mixtures thereof, 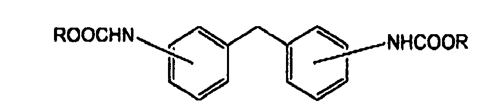 R = C₁-C₆-alkyl, preferably n-butyl 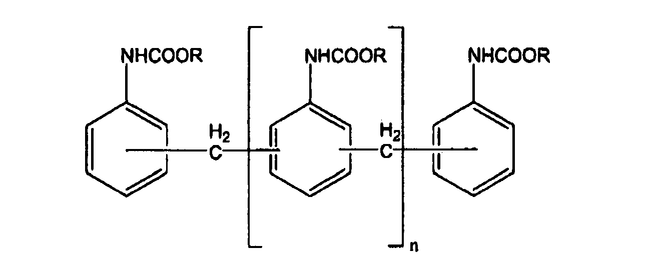 PMDU, R = C₁-C₆-alkyl, preferably n-butyl, [n = 1 - 10]
dialkyl 4,4'-methylene-3,3'-dicarbanilate, dialkyl 2,4'-methylene-3,3'-dicarbanilate, dialkyl 2,2'-methylene-3,3'-dicarbanilate and mixtures thereof, 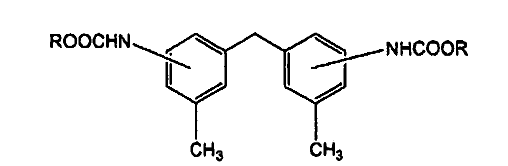 R = C₁-C₆-alkyl, preferably n-butyl
dialkyl 1,2-phenyldicarbamate, dialkyl 1,3-phenyldicarbamate and dialkyl 1,4-phenyldicarbamate and mixtures thereof, 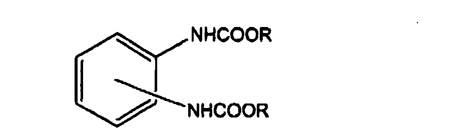 R = C₁-C₆-alkyl, preferably n-butyl
dialkyl 2,4-toluenedicarbamate, dialkyl 2,6-toluenedicarbamate and mixtures thereof, 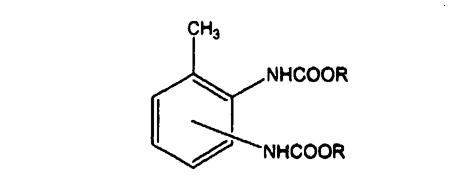 R = C₁-C₆-alkyl, preferably n-butyl
dialkyl 1,6-naphthalenedicarbamate, 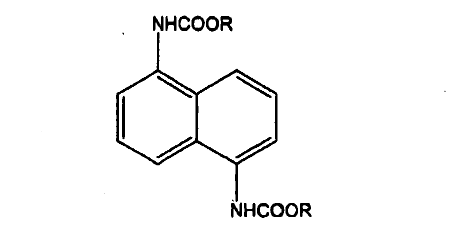 R = C₁-C₆-alkyl, preferably n-butyl
the urethanes corresponding to MXDI and TMXDI, 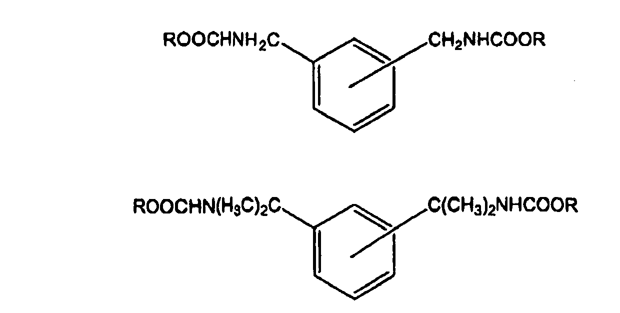 (R = alkyl, preferably n-butyl). is hydrogenated.

12. A process according to at least one of claims 1 to 11,
**characterized in that**
a dialkyl 4,4'-(C₁-C₄)alkanedicarbanilate and/or a 2,4' and/or 2,2' isomer or mixture thereof is hydrogenated.

13. A process according to claim 12,
**characterized in that**
a dibutyl 4,4'-methylenedicarbanilate or an isomer or a mixture thereof is hydrogenated.

14. A process according to at least one of claims 1 to 13,
**characterized in that**
a bridged two-ring starting material is converted into a hydrogenation product having a trans-trans isomer content of < 30%, preferably < 20%, very particularly preferably from 5 to 15%.

15. A process according to at least one of claims 1 to 14 for the hydrogenation of dibutyl 4,4'-methylenedicarbanilate to give dibutyl 4,4'-methylenedicyclohexylcarbamate having a trans-trans isomer content of < 30%, preferably < 20%, particularly preferably from 5 to 15%.

16. A process according to at least one of claims 1 to 15,
**characterized in that**
the hydrogenation is carried out in a solvent or solvent mixture, in particular a solvent comprising alcohols and/or ethers.

17. A process according to claim 16,
**characterized in that**
the alcohol corresponds to the alcohol group of the urethane.

18. A process according to either of claims 16 and 17,
**characterized in that**
n-butanol and tetrahydrofuran are used.

19. A process according to at least one of claims 1 to 18,
**characterized in that**
the dissociation of the hydrogenated urethane in the 3rd step is carried out in the gas phase or liquid phase, with or without catalyst, in the presence or absence of a solvent, continuously or batchwise.

20. A process according to at least one of claims 1 to 19,
**characterized in that**
the dissociation is carried out in a combined dissociation and rectification column.

21. A process according to at least one of claims 1 to 20,
**characterized in that**
the dissociation is carried out in the liquid phase without an additional solvent.

22. A process according to at least one of claims 1 to 21,
**characterized in that**
the dissociation is carried out in the presence of at least one catalyst.

23. A process according to claim 22,
**characterized in that**
from 1 to 2000 ppm of catalyst, based on the volume of the mixture in the dissociation reactor, are used.

24. A process according to at least one of claims 1 to 23,
**characterized in that**
the relatively high molecular weight by-products are discharged from the bottom of the dissociation column.

25. A process according to at least one of claims 1 to 24,
**characterized in that**
the purification of the crude isocyanate taken off from the combined dissociation and rectification column is carried out by vacuum distillation, with first fractions and distillation residues being able to be returned to the combined dissociation and rectification column.

26. A process according to at least one of claims 1 to 25,
**characterized in that**
the dissociation is carried out thermally at a temperature of from 200 to **300°C.**

27. A process according to at least one of claims 1 to 26,
**characterized in that**
the urethane dissociation of the 3rd step is preceded by a rough purification step in which solvents are firstly separated off by nitrogen stripping and further low-boiling secondary components are subsequently at least partly removed by means of a two-stage combination of short path evaporation and thin film evaporation.

28. A process according to at least one of claims 1 to 27,
**characterized in that**
the overall process is carried out fully continuously, partly continuously or batchwise.

29. A process according to at least one of claims 1 to 28 for preparing H₁₂MDI having a trans-trans isomer content of <30%, preferably < 20%, particularly preferably from 5 to 15%, from MDI, comprising essentially the following three steps:
1. Urethanization of the MDI,
2. Hydrogenation of the MDU by means of hydrogen in the presence of a supported catalyst containing ruthenium alone or ruthenium together with at least one metal of transition group I, VII or VIII of the Periodic Table as active metal in an amount of from 0.01 to 20% by weight of total active metals, based on the supported catalyst, applied to a support, where the catalyst support has a BET surface area in the range of from > 30 m²/g to < 70 m²/g and more than 50% of the pore volume of the catalyst support is made up by macropores having a pore diameter greater than 50 nm and less than 50% of the pore volume is made up by mesopores having a pore diameter of from 2 to 50 nm,
3. Dissociation of the hydrogenated urethane to give H₁₂MDI.

30. A process according to at least one of the preceding claims,
**characterized in that**
a sulfphur- and/or phosphorus- and/or chlorine-free starting material is used.

## Revendications

1. Procédé de préparation d'isocyanates cycloaliphatiques à partir des isocyanates aromatiques correspondants, qui présentent un ou plusieurs noyau(x) aromatique(s) et un ou plusieurs groupe(s) isocyanate lié(s) directement et/ou indirectement à un ou à différents noyau(x) aromatique(s), comprenant essentiellement les trois étapes suivantes :
1. uréthanisation de l'isocyanate aromatique,
2. hydrogénation de l'uréthane aromatique avec de l'hydrogène en présence d'un catalyseur supporté, qui contient comme métal actif appliqué sur un support, du ruthénium seul ou conjointement avec au moins un métal des I^{er}, VII^{ième} ou VIII^{ième} groupe(s) secondaire(s) du système périodique, dans une proportion de 0,01 à 20 % en poids de métaux actifs, rapporté au catalyseur supporté, et le support de catalyseur présentant une surface BET couvrant une plage allant de plus de 30 m²/g à moins de 70 m²/g et plus de 50 % du volume poreux du support de catalyseur étant constitués de macropores d'un diamètre supérieur à 50 nm et moins de 50 % de mésopores d'un diamètre de 2 à 50 nm,
3. fission de l'uréthane hydrogéné pour former un isocyanate aliphatique,
avec utilisation d'isocyanates aromatiques choisis parmi les composés suivants :
bis(4-isocyanatophényl)méthane, 2- isocyanatophényl-4'- isocyanatophénylméthane, 2- isocyanatophényl-2'- isocyanatophénylméthane (MDI) et isocyanatophényles pontés avec le groupe méthylène, polynucléaires (PMDI), ainsi que leurs mélanges 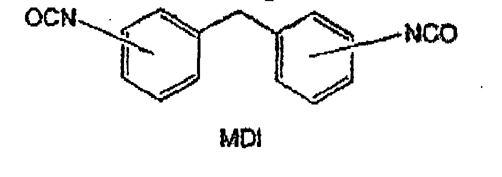 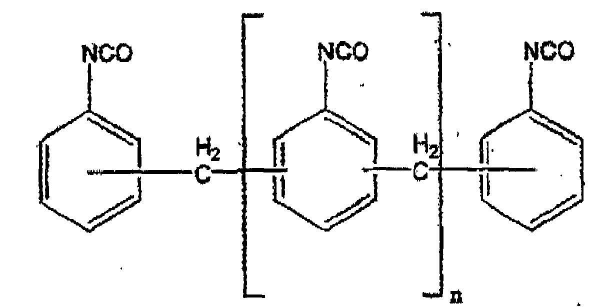 PMDI, n = 1 - 10
4,4'-diisocyanato-3,3'-diméthylphénylméthane, 2,4'-diisocyanato-3,3'-diméthylphénylméthane, 2,2'-diisocyanato-3,3'-diméthylphénylméthane, ainsi que leurs mélanges, 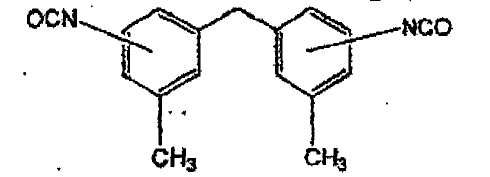
1,2-diisocyanatobenzène, 1,3 diisocyanatobenzène, et 1,4 diisocyanatobenzène, ainsi que leurs mélanges,
2,4-diisocyanatotoluène, 2,6- diisocyanatotoluène, ainsi que leurs mélanges, 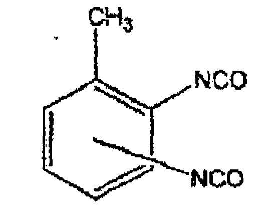
1,6- diisocyanatonaphtaline, 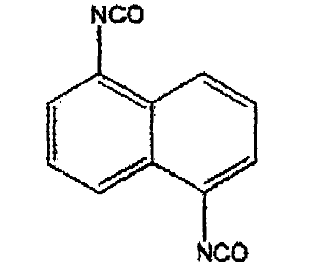 MXDI et TMXDI,

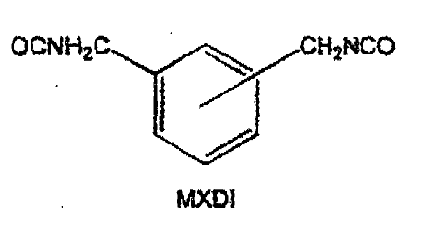

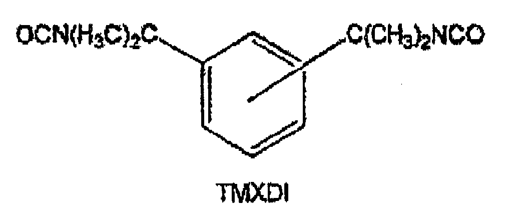

2. Procédé selon la revendication 1,
**caractérisé en ce que**
l'uréthanisation dans la première étape est effectuée en continu ou en discontinu, en présence ou en l'absence d'un solvant ou d'un mélange de solvants, en présence ou en l'absence d'un catalyseur, à des températures situées entre 20 et 160 °C.

3. Procédé selon au moins une des revendications 1 à 2,
**caractérisé en ce que**
l'uréthanisation est effectuée dans un alcool, en particulier dans le n-butanol.

4. Procédé selon au moins une des revendications 1 à 3,
**caractérisé en ce que**
le métal actif appliqué sur le catalyseur de la deuxième étape présente une profondeur de pénétration dans le support située dans une plage de 20 à 500 µm, en particulier de 25 à 250 µm.

5. Procédé selon au moins une des revendications 1 à 4,
**caractérisé en ce que**
le rapport de la surface du métal actif, déterminée par chimisorption par pulsations de CO, et de la surface du support de catalyseur, déterminée selon BET, est supérieur à 0,01, et en particulier de 0,03 à 0,3.

6. Procédé selon au moins une des revendications 1 à 5,
**caractérisé en ce que**
la matière support est choisie dans la série des oxydes et silicates cristallins et amorphes, en particulier choisis dans la série Al₂O₃, SiO₂, TiO₂ ZrO₂, MgO, ZnO et alumosilicates.

7. Procédé selon au moins une des revendications 1 à 6,
**caractérisé en ce que**
le support de catalyseur présente une surface BET de 32 à 67 m²/g, la profondeur de pénétration des métaux actifs est de 50 à 200 µm et la quantité de Ru est de 0,2 à 3 % en poids, rapporté au catalyseur, et au moins 55 % du volume poreux du support de catalyseur sont constitués de macropores et moins de 45 % de mésopores.

8. Procédé selon au moins une des revendications 1 à 7,
**caractérisé en ce qu'**
on effectue l'hydrogénation dans un réacteur d'hydrogénation en suspension ou à lit fixe fonctionnant en continu ou en discontinu, à une température située entre 20 à 250 °C, en particulier de 50 à 150 °C, de manière particulièrement préférable de 70 à 120 °C et avec une pression partielle d'hydrogène de 1 à 30 MPa, en particulier de 3 à 15 MPa.

9. Procédé selon au moins une des revendications 1 à 8,
**caractérisé en ce qu'**
on effectue l'hydrogénation dans un réacteur à lit fixe, en particulier dans un réacteur à faisceau tubulaire, selon le procédé du lit ruisselant.

10. Procédé selon au moins une des revendications 1 à 9,
**caractérisé en ce qu'**
on utilise un catalyseur supporté dont le métal actif, le ruthénium, a été appliqué sur un support par aspersion de ce dernier avec une solution de sel de ruthénium diluée, en particulier une solution de nitrosylnitrate de ruthénium à une température d'au moins 80 °C, suivie d'un recuit et d'une activation du catalyseur par réduction dans un gaz contenant de l'hydrogène.

11. Procédé selon au moins une des revendications 1 à 10,
**caractérisé en ce qu'**
on hydrogène des composés choisis parmi le 4,4'-méthylènedicarbanilate de dialkyle, le 2,4' -méthylènedicarbanilate de dialkyle, le 2,2'- méthylènedicarbanilate de dialkyle, et des carbanilates d'alkyle pontés avec le groupe méthylène, polynucléaires, (PMDU), ainsi que leurs mélanges, 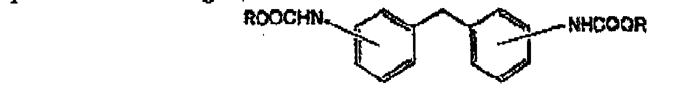 R = alkyle en C₁- C₆, de préférence n-butyle, 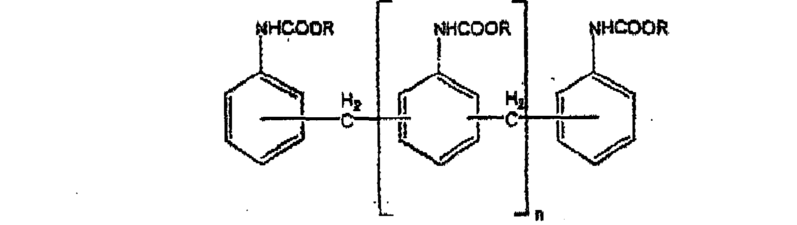 PMDU, R = alkyle en C₁ - C₆, de préférence n-butyle (n = 1 - 10) 4,4'-méthylèn-3,3'-dicarbanilate de dialkyle, 2,4'- méthylèn-3,3'-dicarbanilate de dialkyle,
2,2'- méthylèn-3,3'-dicarbanilate de dialkyle, ainsi que leurs mélanges. 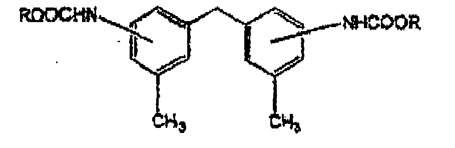 R = alkyle en C₁ - C₆, de préférence n-butyle
1,2-phényldicarbamate de dialkyle, 1,3- phényldicarbamate de dialkyle, et 1,4- phényldicarbamate de dialkyle, ainsi que leurs mélanges, 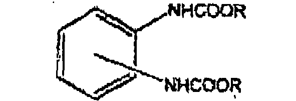 R = alkyle en C₁- C₆, de préférence n-butyle
2,4-toluènedicarbamate de dialkyle, 2,6- toluènedicarbamate de dialkyle, ainsi que leurs mélanges 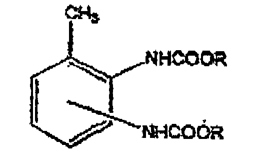 R = alkyle en C₁ - C₆, de préférence n-butyle
1,6-naphtalénedicarbamate de dialkyle, 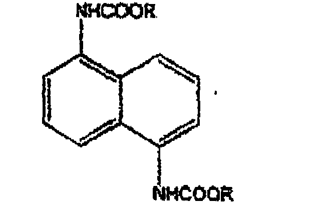 R = alkyle en C₁- C₆, de préférence n-butyle,
les uréthanes correspondant aux MXDI et TMXDI cités, 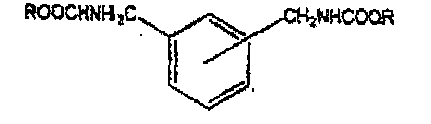 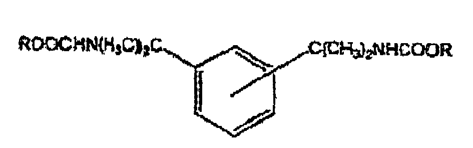 (R = alkyle en C₁ - C₆, de préférence n-butyle)

12. Procédé selon au moins une des revendications 1 à 11,
**caractérisé en ce qu'**
on hydrogène un 4,4'-alcane (en C₁ - C₄) -dicarbanilate de dialkyle et/ou un isomère 2,4' et /ou 2,2', ou leurs mélanges.

13. Procédé selon la revendication 12,
**caractérisé en ce qu'**
on hydrogène un 4,4'-méthylènedicarbanilate de dibutyle ou un isomère ou un mélange.

14. Procédé selon au moins une des revendications 1 à 13,
**caractérisé en ce qu'**
on prépare, à partir de produits de départ binucléaires pontés, des produits d'hydrogénation présentant une proportion d'isomère trans-trans < 30 %, de préférence < 20 %, de manière tout particulièrement préférable de 5 à 15 %.

15. Procédé selon au moins une des revendications 1 à 14,
pour l'hydrogénation de 4,4'-méthylènedicarbanilate de dibutyle en 4,4'-méthylènedicyclohexylcarbamate de dibutyle avec une proportion d'isomère trans-trans < 30 %, de préférence < 20 %, particulièrement préférablement de 5 à 15 %.

16. Procédé selon au moins une des revendications 1 à 15,
**caractérisé en ce que**
l'hydrogénation est effectuée dans un solvant ou un mélange de solvants, en particulier constitué d'alcools et/ou d'éthers.

17. Procédé selon la revendication 16,
**caractérisé en ce que**
l'alcool correspond au reste alcool de l'uréthane.

18. Procédé selon les revendications 16 ou 17,
**caractérisé en ce que**
on utilise le n-butanol et le tétrahydrofurane.

19. Procédé selon au moins une des revendications 1 à 18,
**caractérisé en ce que**
la fission des uréthanes hydrogénés a lieu, au cours de la troisième étape, en phase gazeuse ou en phase liquide, avec ou sans catalyseur, en présence ou en l'absence de solvants, de manière continue ou discontinue.

20. Procédé selon au moins une des revendications 1 à 19,
**caractérisé en ce que**
la fission a lieu dans une colonne de fractionnement et de rectification combinée.

21. Procédé selon au moins une des revendications 1 à 20,
**caractérisé en ce que**
la fission a lieu en phase liquide sans solvant supplémentaire.

22. Procédé selon au moins une des revendications 1 à 21,
**caractérisé en ce que**
la fission a lieu en présence d'au moins un catalyseur.

23. Procédé selon la revendication 22,
**caractérisé en ce qu'**
on utilise 1 à 2 000 ppm de catalyseur, rapporté au volume du mélange dans le réacteur de fission.

24. Procédé selon au moins une des revendications 1 à 23,
**caractérisé en ce que**
les sous-produits de poids moléculaire supérieur sont évacués du fond de la colonne de fractionnement.

25. Procédé selon au moins une des revendications 1 à 24,
**caractérisé en ce que**
la purification de l'isocyanate brut retiré de la colonne de fractionnement et de rectification combinée a lieu par distillation sous vide, les fractions de tête et les résidus de distillation pouvant être recyclés dans la colonne de fractionnement et de rectification combinée.

26. Procédé selon au moins une des revendications 1 à 25,
**caractérisé en ce que**
la fission est effectuée par voie thermique à une température de 200 à 300 °C.

27. Procédé selon au moins une des revendications 1 à 26,
**caractérisé en ce que**
la fission de l'uréthane de la troisième étape est précédée d'une étape de pré-purification grossière, dans laquelle on sépare d'abord le solvant par strippage d'azote, et on élimine ensuite, à l'aide d'une combinaison en deux étapes d'évaporation courte et d'évaporation d'une couche mince, d'autres composants secondaires de bas point d'ébullition.

28. Procédé selon au moins une des revendications 1 à 27,
**caractérisé en ce que**
l'ensemble du procédé est mis en oeuvre d'une manière entièrement continue, partiellement continue ou discontinue.

29. Procédé selon au moins une des revendications 1 à 28,
pour la préparation de H₁₂ MDI présentant une proportion d'isomère trans-trans < 30 %, de préférence < 20 % particulièrement préférablement de 5 à 15 % à partir de MDI, comprenant essentiellement les trois étapes suivantes :
1. uréthanisation du MDI,
2. hydrogénation du MDU avec de l'hydrogène en présence d'un catalyseur supporté, qui contient comme métal actif appliqué sur un support du ruthénium, seul ou conjointement avec au moins un métal des I^{er}, VII^{ième} ou VIII^{ième} groupe(s) secondaire(s) du système périodique, dans une proportion de 0,01 à 20 % en poids de métaux actifs, rapporté au catalyseur supporté, et le support de catalyseur présentant une surface BET couvrant une plage allant de plus de 30 m²/g à moins de 70 m²/g et plus de 50 % du volume poreux du support de catalyseur étant constitués de macropores d'un diamètre supérieur à 50 nm et moins de 50 % de mésopores d'un diamètre de 2 à 50 nm
3. fission de l'uréthane hydrogéné pour obtenir H₁₂ MDI.

30. Procédé selon une des revendications précédentes,
**caractérisé en ce qu'**
on utilise une matière de départ exempte de soufre et/ ou de phosphore et/ou de chlore.
